# EUROPEAN PATENT SPECIFICATION

(11) **EP 3 634 409 B1**
(45) Date of publication and mention of the grant of the patent: **07.04.2021**
(21) Application number: 18730033.0
(22) Date of filing: 10.05.2018
(51) Int. Cl.: A61K 31/4439, A61P 25/00, A61P 15/00, A61P 3/00, A61P 43/00, A61P 5/00

(54) **OREXIN RECEPTOR ANTAGONISTS**
OREXINREZEPTORANTAGONISTEN
ANTAGONISTES DES RÉCEPTEURS DE L'OREXINE

(30) Priority: 10.05.2017 GB 201707499
(43) Date of publication of application: 15.04.2020
(73) Proprietor: BenevolentAI Bio Limited, London W1T 5HD (GB)
(72) Inventor: SHEPPARD, David, Wickhambrook Suffolk CB8 8UN (GB); ANDREOTTI, Daniele, 37135 Verona (IT)
(74) Representative: Gill Jennings & Every LLP
(86) International application number: PCT/GB2018/051256
(87) International publication number: WO 2018/206956

(56) References cited:
- WO-A1-2011/050198
- WO-A2-2016/100157
- MICHAEL A. LETAVIC ET AL: "Novel Octahydropyrrolo[3,4- c ]pyrroles Are Selective Orexin-2 Antagonists: SAR Leading to a Clinical Candidate", JOURNAL OF MEDICINAL CHEMISTRY, vol. 58, no. 14, 8 July 2015 (2015-07-08), pages 5620-5636, XP055493378, ISSN: 0022-2623, DOI: 10.1021/acs.jmedchem.5b00742

## Description

This invention relates to orexin receptor antagonists, pharmaceutical compositions comprising the antagonists, methods of making the antagonists, their use for the modulation of the orexin receptor, and to use of the antagonists as medicaments, in particular for treating diseases, disorders, or conditions mediated by orexin receptor activity.

Orexin (or hypocretin) signalling is mediated by two receptors and two peptide agonists. The two orexin peptides (orexin A and orexin B), bind to two high affinity G protein-coupled receptors (GPCRs), the orexin-1 and orexin-2 receptors (Sakurai T. et al., Cell, 1998, 92, 573-585). Orexin B binds preferentially to orexin-2 receptors, whereas orexin A binds with equal affinity to orexin-1 and orexin-2 receptors. Orexin A and orexin B (the orexins) are cleavage products of the same gene, prepro orexin. In the central nervous system neurons expressing prepro-orexin, the precursor from which orexin is produced, are found in the perifornical nucleus, the dorsal hypothalamus and the lateral hypothalamus. Orexinergic cells in these nuclei project to many areas of the brain, extending rostrally to the olfactory bulbs and caudally to the spinal cord.

The orexin system acts as an integrator of numerous physiological functions, including sleep/arousal states, sensory, locomotion, cognition, energy homeostasis, endocrine and visceral functions (reviewed by Li et al., British Journal of Pharmacology (2014) 171, 332-350).

Orexin signalling is essential for the stability of the arousal state. It is thought that the orexin system acts as a regulator of behaviour states by modulating the arousal threshold so that appropriate wakefulness can be maintained to cope with fluctuations in the external and internal environments. A lack of orexin signalling is associated with narcolepsy, a disorder characterised by uncontrollable sleep episodes and hypersomnolence (Nishino et al., Lancet: 355, 39-40. 2000)). Dual Orexin 1 and 2 receptor antagonists (DORAs) have proven efficacy in human patients suffering from insomnia, with Suvorexant (Belsomra) the first Orexin dual antagonist approved as an insomnia treatment. However evidence is mounting to suggest that these DORAs function predominantly through OX2R antagonism. For example, OX1R knock-out mice exhibit a subtle, barely detectable sleep phenotype, whereas OX2R knock-out mice show profound hyper somnolence (Kisanuki et al., Sleep 23, A91. 2000). Furthermore, in dogs OX2R mutations alone are responsible for the narcoleptic phenotype (Lin et al., Cell 98:365-376. 1999). Questions have been raised in the literature as to whether OX1R antagonism may actually have detrimental effects in humans, causing an imbalance in the normal REM/NREM sleep architecture and given the brainstem localisation of OX1R, particularly in the locus coeruleus, OX1R antagonists may act to attenuate vigilance state boundaries, effectively diminishing the threshold for sleep-state transitions (Dugovic et al., Front Neurosci. 8:28. 2014). The evidence suggests that a selective OX2R antagonist may provide safer, more efficacious, treatments than DORAs in humans. Disorders of the sleep-wake cycle are, therefore, likely targets for orexin receptor (especially orexin-2 receptor) antagonist therapy. Examples of such disorders include sleep-wake transition disorders, insomnia, restless legs syndrome, jet-lag, disturbed sleep, and sleep disorders secondary to neurological disorders (for example, manias, depressions, manic depression, schizophrenia, and pain syndromes, such as fibromyalgia, neuropathic pain).

The orexin system also interacts with brain dopamine systems. Intracerebroventricular (i.c.v.) injections of orexins in mice increase locomotor activity, grooming and stereotypy. These effects are reversed by administration of D₂ dopamine receptor antagonists (Nakamura et al., Brain Research, 2000, 873(1),181-7). Therefore, orexin-receptor antagonists may be useful to treat various neurological disorders, for example, Parkinson's disease, Tourette's syndrome, anxiety, delirium and dementias.

There is evidence for a role for orexin in the pathogenesis of Alzheimer's disease (Kang et al, Science Express, 2009, 1-10). Brain interstitial fluid levels of amyloid-beta were demonstrated to fluctuate diurnally in both humans and rodents with sleep deprivation in rodents leading to significant increases in brain interstitial fluid levels of amyloid-beta. Infusion of a dual orexin antagonist in rodents suppressed interstitial levels of amyloid-beta and abolished the natural diurnal variation of amyloid-beta. The reduction of interstitial fluid amyloid-beta levels is correlated with reduced amyloid plaque formation, a hallmark of Alzheimer's disease, and consequently the regulation of sleep time could potentially inhibit amyloid-beta aggregation and slow the progression of Alzheimer's disease.

Orexin neurons project to many regions of the brain associated with reward function (T. Sakurai, *supra*)*.* Several studies have demonstrated a direct role for orexin in the re-instatement of opioid-, cocaine-, alcohol-, and nicotine-seeking behaviour (Harris et al, 2005, Nature 437: 556-559; Aston-Jones et al, 2008, Neuropharmacology 56 (Suppl. 1): 112-121; Lawrence et al, 2006, Br J Pharmacol 148: 752-759; Plaza-Zabala et al, 2010, J Neurosci 30, 2300-2310). Thus, orexin receptor antagonists are likely to be useful for the treatment of drug addiction, and alcoholism.

Intracisternal or intraventromedial hypothalamus injections of orexin-A stimulate gastric acid secretion by activating the vagal system through orexin-1 receptors (Takahashi et al., Biochem Biophys Res Commun, 1999, 254: 623-627; Yamada et al., Neurosci Lett, 2005, 376: 137-142; Eliassi et al., J Neuroendocrinol, 2009, 21: 177-182; Kermani and Eliassi, Neuroscience, 2012, 226: 81-88). Orexin-A can modify gastrointestinal motility, including gastric emptying, gastric interdigestive motility (Naslund et al., Am J Physiol Gastrointest Liver Physiol, 2002, 282: G470-G479; Ehrstrom et al., J Clin Endocrinol Metab, 2005, 90: 2370-2377; Ehrstrom et al., Regul Pept, 2005, 132: 9-16; Bulbul et al., Peptides, 2010, 31: 2118-2122), and enteric peristalsis (Satoh et al., Neuropharmacology, 2006, 51: 466-473), as well as colonic motility (Kirchgessner and Liu, Neuron, 1999, 24: 941-951; Nozu et al., Neurosci Lett, 2011, 498: 143-146). Orexin receptor antagonists are therefore potential treatments for ulcers, irritable bowel syndrome, diarrhoea and gastroesophageal reflux.

Body weight may also be affected by orexin-mediated regulation of appetite and metabolism (T. Sakurai et al., Cell, 1998, 92(4), 573-585; T. Sakurai, Reg. Pept., 1999, 85(1), 25-30). Various findings suggest that orexinergic neurons maintain a proper balance between energy intake or storage and expenditure initially by monitoring the nutritional state of the body in real time. Orexins also regulate energy intake or storage and expenditure by modulating the feeding and energy homeostatis-related circuits. Orexinergic neurons in the LH have a prominent role in sending the steady-state levels of physiologically relevant metabolites and integrate this information into a coherent value that is conveyed to arousal centres (Adamantidis and de Lecea, Trends Endocrinol Metab., 2008, 19: 362-370; 2009; Carter et al., Current Opin Pharmacol 2009, 9: 39-45). In addition to a direct interaction with feeding circuits and with humoral signals affecting feeding, orexinergic neurons have a significant regulatory role in the brain reward system, and particularly for palatable food. The orexin-1 receptor has been shown to mediate food motivation and reward-based feeding behaviour in mice and rats (Sharf et al., Brain Res, 2010, 1314: 130-138; Choi et al., Neuroscience, 2010, 167: 11-20). Orexin receptor antagonists, therefore, are likely to be useful in treatment of overweight or obesity, and conditions related to overweight or obesity, such as insulin resistance, type II diabetes, hyperlipidemia, gallstones, angina, hypertension, breathlessness, tachycardia, infertility, sleep apnoea, back and joint pain, varicose veins and osteoarthritis.

Orexin-deficient mice exhibit lower arterial blood pressure, heart rate and sympathetic tone (Kayaba et al., 2003, Am J Physiol Regul Integr Comp Physiol 285: R581-R593). Orexin administered i.c.v., intracisternally or intrathecally increases the mean arterial pressure, heart rate, renal sympathetic nerve activity and plasma catecholamine or vasopressin levels (Samson et al., Brain Res, 1999, 831: 248-253; Samson et al., Sleep Med Rev, 2005, 9: 243-252; Shirasaka et al., Am J Physiol, 1999, 277 (6 Pt 2): R1780-R1785; Shirasaka et al., Regul Pept, 2002, 104: 91-95; Matsumura et al., Hypertension, 2001, 37: 1382-1387; Hirota et al., Brain Res, 2003, 981: 143-150). These effects that are blocked or attenuated by the OX1 receptor antagonist SB-334867 (Hirota *et al.,* 2003, *supra*; Shahid et al., Br J Pharmacol, 2011, 162: 961-973). Thus, orexin receptor antagonists may be useful for treatment of hypertension, tachycardia and other arrhythmias, angina pectoris and acute heart failure.

It has been suggested that orexinergic neurons are involved in sleep apnoea syndrome, as patients show increased plasma levels of orexin-A (Igarashi et al., Chest, 2003, 124: 1381-1385; Busquets et al., Respiration, 2004, 71: 575-579). Hypercapnia and associated reflexes may increase the activity of orexinergic neurons during sleep, facilitating microarousals and a cascade of sympathetic activity that results in elevated blood pressure during the night. Thus, orexin receptor antagonists could be used to prevent these peaks of blood pressure in mild sleep apnoea.

The role of the orexinergic system in stress responses has been well established. The term 'stress' means a subjective state perceiving or anticipating the adverse disturbances in surroundings, which further activates various stress mediators to elicit proper responses (Joels, Epilepsia, 2009, 50: 586-597). Activation of orexinergic neurons results in some stress-like effects. Orexin can activate the hypothalamic-pituitary-adrenal (HPA) axis, including corticotrophin releasing factor (CRF), adrenocorticotropic hormone (ACTH) and corticosterone, stimulate stress-related behaviours, like grooming and chewing of inedible material, and enhance the activation of the monoamine system in a stress-like manner (Berridge et al., Brain Res, 2010, 1314: 91-102). A link between orexin and stress-related pathologies, such as panic disorder, has been established (Johnson et al., Nat Med, 2010, 16: 111-115; Lungwitz et al., Physiol Behav, 2012, 107: 726-732). Panic attacks involve activation of the HPA axis and the autonomic system. Intrahypothalamic administration of an RNAi to orexin or an OX1 receptor antagonist has been shown to block these panic responses in rats injected with sodium lactate, and elevated levels of orexin-A have been detected in humans with panic disorder (Johnson *et al.,* 2010, *supra*)*.* Orexin receptor antagonists may be useful for the treatment of stress, anxiety and panic disorders.

Identification of orexin receptor antagonists is of great significance in the development of therapeutic agents for the treatment of a wide variety of disorders that are mediated through orexin receptors.

Various small-molecule orexin receptor modulators have been reported, including: N-aroyl cyclic amine derivatives (WO 2003/002561), ethylene diamine derivatives (WO 2003/051872), sulfonylamino-acetic acid derivatives (WO 2004/033418), N-aryl acetyl cyclic amine derivatives (WO 2004/041791), diazepan derivatives (WO 2007/126935), amidoethylthioether derivatives (WO 2007/126934), 2-substituted proline bis-amide derivatives (WO 2008/008551), bridged diazepan derivatives (WO 2008/008517), substituted diazepan derivatives (WO 2008/008518; US 2008/0132490, WO 2009/058238), oxo bridged diazepan derivatives (WO 2008/143856), 1,2-diamido ethylene derivatives (WO 2009/022311), heteroaryl derivatives (WO 2009/0163485), methyl substituted piperidinyl derivatives (WO 2009/124956), N,N-disubstituted-1 ,4-diazepane derivatives (Cox et al., Bioorganic & Medicinal Chemistry Letters, 2009, 19(11), 2997-3001), Orexin/Hypocretin receptor ligands (Boss, et al., Journal of Medicinal Chemistry, 2009, 52(4), 891-903) 3,9-diazabicyclo[4.2.1]nonanes (Coleman et al., Bioorganic & Medicinal Chemistry Letters, 2010, 20(14), 4201-4205), the dual orexin receptor antagonist, [(7R)-4-(5-Chloro-1 ,3-benzoxazol-2-yl)-7 -methyl-1 ,4- diazepan-1-yl][5-methyl-2-(2H-1 ,2,3-triazol-2-yl)phenyl]methanone, (Cox, et. al., Journal of Medicinal Chemistry, 2010 53(14) 5320-5332), pyridazine carboxamide derivatives (WO 2010/051238), 2,5- disubstituted benzamide derivatives (WO 2010/051237), isonicotinamides (WO 2010/051236), heterocyclylbenzoylpiperazines derivatives (WO 2010/48012), substituted diazepane derivatives (WO 2010/048017), substituted pyrrolidine derivatives (WO 2010/048014), triazolylbenzoylpiperidine derivatives (WO 2010/048010), triazolylbenzoylmorpholine derivatives (WO 2010/048013), conformationally restrained N,N disubstituted 1 ,4-diazapane derivatives (Coleman et al., Bioorganic & Medicinal Chemistry Letters, 2010, 20(7), 2311- 2315), tripyridyl carboxamide derivatives (WO 2010/017260), imidazopyridylmethyl substituted piperidine derivatives (WO 2010/072722), imidazopyrazine substituted piperidine derivatives (US 2010/160344; US 2010/0160345; WO 2010/060472), N-{[(1 R,4S,6R)-3-(2-pyridinylcarbonyl)-3-azabicyclo[4.1.0]hept-4-yl]methyl}-2-heteroarylamine derivatives (WO 2010/063663), N-{[(1 S,4S,6S)-3-(2-pyridinylcarbonyl)-3-azabicyclo[4.1.0]hept-4-yl]methyl}-2-heteroarylamine derivatives (WO 2010/063662), imidazopyrimidine derivatives (WO 2010/060471), imidazopyrazine derivatives (WO 2010/060470), disubstituted octahydropyrrolo[3,4-c]pyrrole derivatives (WO 2012/145581).

There remains a need, however, for potent orexin receptor modulators, in particular orexin receptor antagonists.

According to the invention, there is provided a compound of Formula (I):

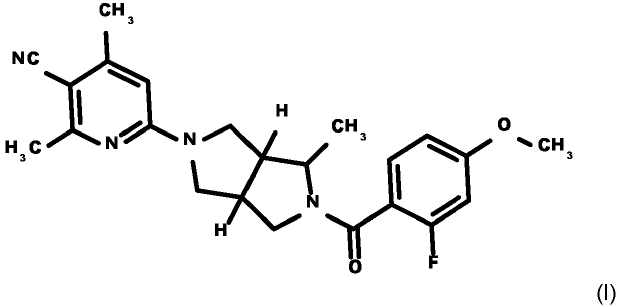

Compounds of the invention include compounds of Formula (I) and salts thereof, polymorphs, and isomers thereof (including optical, geometric and tautomeric isomers) as hereinafter defined and isotopically-labelled compounds of Formula (I).

In some embodiments, the central hydrogen atoms (shown in the above structural formula) on the methylated octahydropyrrolo[3,4-c]pyrrole group are in a *cis* position.

In particular embodiments, the compound of Formula (I) is a compound of Formula (I)(a):

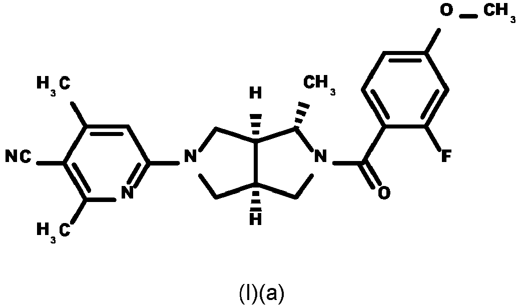

6-[(3aS,4S,6aR)-5-(2-Fluoro-4-methoxybenzoyl)-4-methyl-octahydropyrrolo[3,4-c]pyrrol-2-yl]-2,4-dimethylpyridine-3-carbonitrile

Compounds of the invention include compounds of Formula (I)(a) and salts thereof, polymorphs, and isomers thereof (including optical, geometric and tautomeric isomers) as hereinafter defined and isotopically-labelled compounds of Formula (I)(a).

Compounds of the invention have been found to have surprisingly high potency as orexin receptor antagonists, in particular as selective OX2 receptor antagonists. We have found that incorporation of the methyl substituent at position 4 of the octahydropyrrolo[3,4-c]pyrrole group provides compounds of the invention with an unexpectedly high increase in potency as an orexin receptor antagonist compared with a corresponding desmethylated compound.

In view of their surprisingly high potency as orexin receptor antagonists, compounds of the invention are expected to be particularly effective in the treatment of diseases, disorders or conditions mediated by orexin receptor activity, especially those associated with OX2 receptor activity. Compounds of the invention may be more effective than conventional compounds in the treatment of such diseases, disorders or conditions at similar doses to the conventional compounds. Altematively or additionally, compounds of the invention may be as effective as conventional compounds at lower doses than the conventional compounds but have fewer, or reduced side effects.

There is also provided according to the invention a pharmaceutical composition comprising a compound of formula (I) or (I)(a), or a pharmaceutically acceptable salt thereof, and a pharmaceutically acceptable excipient.

There is further provided according to the invention a compound or composition of the invention for use as a medicament.

The invention also provides a compound or composition of the invention for use in treating a disease, disorder or condition mediated by orexin receptor activity.

According to the invention there is also provided use of a compound or composition of the invention in the manufacture of a medicament for treating a disease, disorder or condition mediated by orexin receptor activity.

There is further provided according to the present disclosure a method of treating a disease, disorder or condition mediated by orexin receptor activity, which comprises administering an effective amount of a compound of Formula (I) or (I)(a), or a pharmaceutically acceptable salt thereof, to a subject in need thereof.

The invention also provides a method of producing a compound of Formula (I), which comprises reacting a compound of formula (V) and a compound of formula (XVI), to produce the compound of Formula (I): 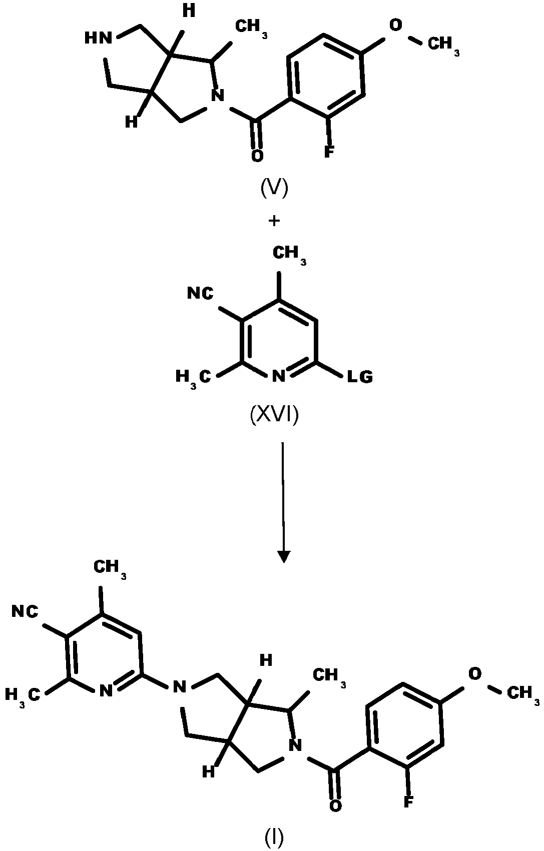 where LG is a leaving group.

Examples of suitable leaving groups include: a chloro, bromo, mesylate or tosylate group. In particular embodiments, LG is chloro.

In some embodiments, the central hydrogen atoms on the methylated octahydropyrrolo[3,4-c]pyrrole group of the compound of Formula (V) are in a *cis* position, thereby producing a compound of Formula (I) with corresponding hydrogen atoms in a *cis* position.

In particular embodiments, there is provided a method of producing a compound of Formula (I)(a), which comprises reacting a compound of formula (V)(a) and a compound of formula (XVI), to produce the compound of Formula (I)(a): 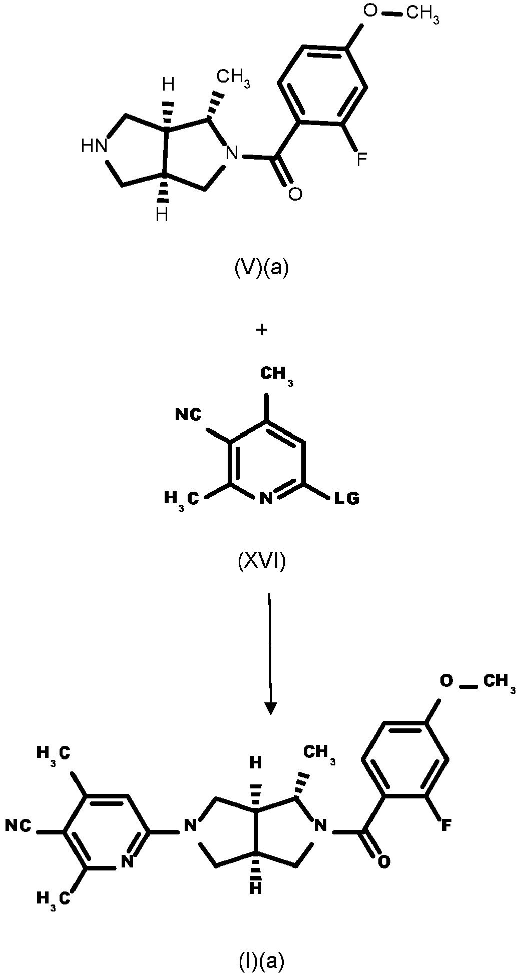 where LG is a leaving group, such as a chloro, bromo, mesylate, or tosylate group, in particular chloro.

Typically, the compound of Formula (V) or (V)(a) is reacted with the compound of Formula (XVI) in the presence of a base in a solvent.

In some embodiments, the compound of Formula (V) or (V)(a) is reacted with the compound of Formula (XVI) in the presence of a suitable base such as CS₂CO₃ or K₂CO₃ in a polar solvent such as DMF, DMSO or EtOH. In particular embodiments, the base is K₂CO₃, and the solvent is EtOH or DMSO, and the reaction is carried out at an elevated temperature, preferably between 70°C and 80°C. In particular, the base is K₂CO₃, and the solvent is EtOH or DMSO, and the reaction is carried out at an elevated temperature, preferably between 70°C and 80°C.

In some embodiments the compound of Formula (XVI) is 6-Chloro-4,6-dimethylnicotinonitrile (i.e. LG is chloro).

In some embodiments, methods of the invention for producing a compound of Formula (I) further comprise producing a compound of Formula (V) by deprotection of a compound of Formula (IV): 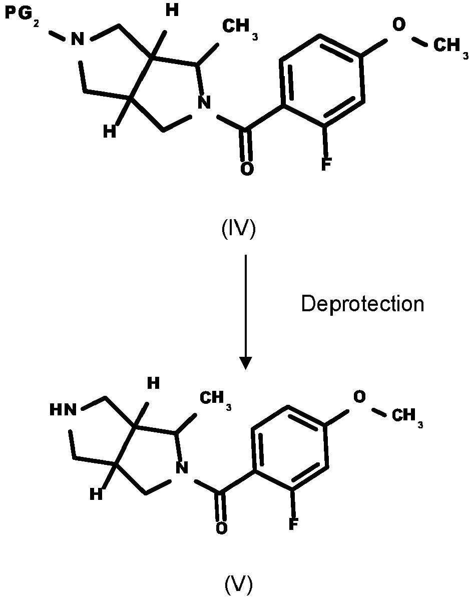 where PG₂ is an amine-protecting group

In some embodiments, the central hydrogen atoms on the methylated octahydropyrrolo[3,4-c]pyrrole group of the compound of Formula (IV) are in a *cis* position, thereby producing a compound of Formula (V) with corresponding hydrogen atoms in a *cis* position.

In particular embodiments, methods of the invention for producing a compound of Formula (I)(a) further comprise producing a compound of Formula (V)(a) by deprotection of a compound of Formula (IV)(a): 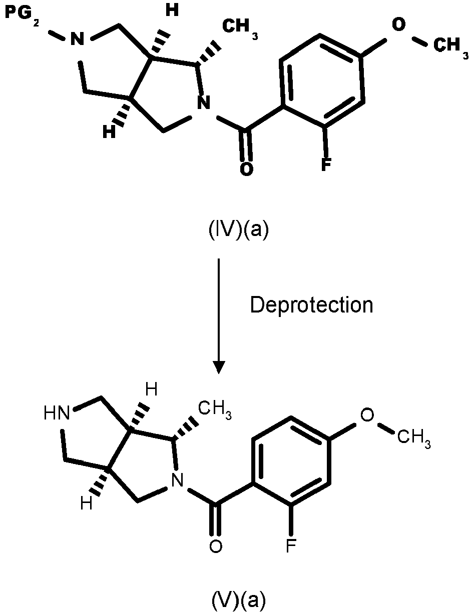 where PG₂ is an amine-protecting group.

In some embodiments, (in particular where PG₂ is benzyl) the deprotection is carried out by transfer or palladium-catalyzed hydrogenation. In particular embodiments, palladium catalyzed hydrogenation may be carried out using 10% palladium on charcoal in EtOH under an atmosphere of hydrogen at room temperature.

In some embodiments, methods of the invention for producing a compound of Formula (I) further comprise producing a compound of Formula (IV) by reacting a compound of Formula (III) with 2-fluoro-4-methoxybenzoic acid to form the compound of Formula (IV): 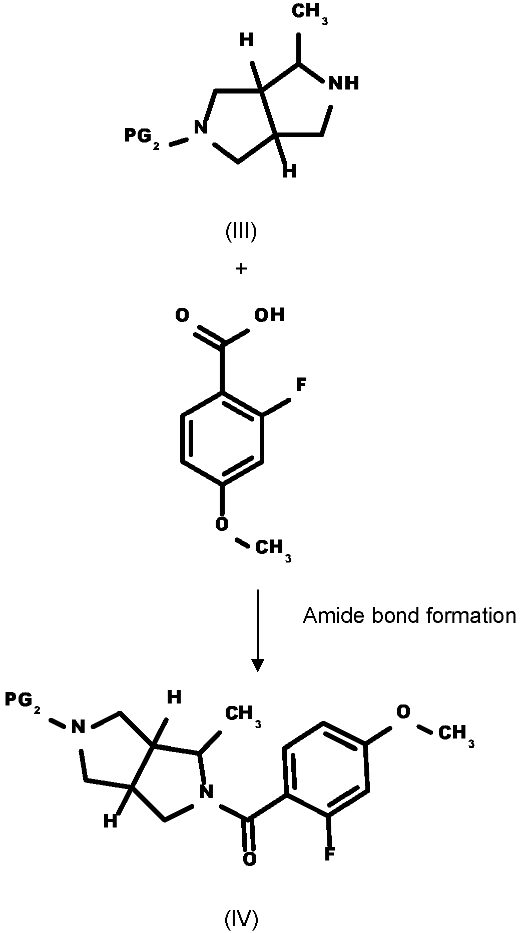 where PG₂ is an amine-protecting group.

In some embodiments, the central hydrogen atoms on the methylated octahydropyrrolo[3,4-c]pyrrole group of the compound of Formula (III) are in a *cis* position, thereby producing a compound of Formula (IV) with corresponding hydrogen atoms in a *cis* position.

In particular embodiments, methods of the invention for producing a compound of Formula (I)(a) further comprise producing a compound of Formula (IV)(a) by reacting a compound of formula (III)(a) with 2-fluoro-4-methoxybenzoic acid to form the compound of Formula (IV)(a): 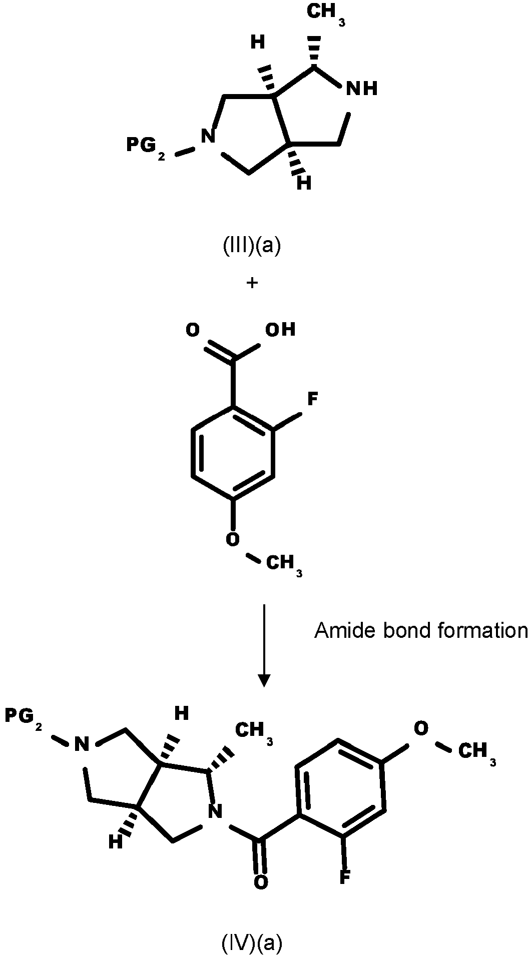 where PG₂ is an amine-protecting group.

In some embodiments, the compound of Formula (III) or (III)(a) is reacted with 2-fluoro-4-methoxybenzoic acid in the presence of a coupling reagent, such as HATU or T3P, and an organic base, such as Et₃N or DIPEA, in an aprotic solvent, such as MeCN or DMF, at a temperature from 0-100°C, such as room temperature. Alternatively, in some embodiments, 2-fluoro-4-methoxybenzoic acid is converted *in situ* to 2-fluoro-4-methoxybenzoyl chloride, by treatment with a suitable reagent, such as SOCl₂ or PCl₃, in a non polar solvent, such as toluene, at elevated temperature, followed by reaction with a compound of Formula (III) or (III)(a), in the presence of a suitable organic base, such as Et₃N or DIPEA, in an aprotic solvent, such as DCM or MeCN, typically at a temperature from 0°C to room temperature.

In particular embodiments, the compound of Formula (III) or (III)(a) is reacted with 2-fluoro-4-methoxybenzoic acid in the presence of T3P and Et₃N in MeCN at a temperature from 0°C to room temperature.. Alternatively, 2-fluoro-4-methoxybenzoic acid is treated with SOCl₂, in toluene at 80°C to prepare 2-fluoro-4-methoxybenzoyl chloride *in situ,* followed by reaction with a compound of Formula (III) or (III)(a) in the presence of Et₃N in DCM at room temperature.

In some embodiments, methods of the invention for producing a compound of Formula (I) further comprise producing a compound of Formula (III) by reduction of a compound of Formula (II): 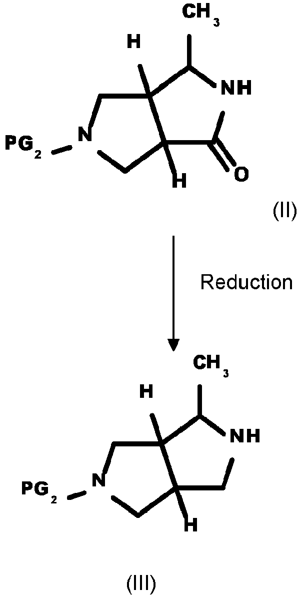 where PG₂ is an amine-protecting group.

In some embodiments, the central hydrogen atoms on the methylated octahydropyrrolo[3,4-c]pyrrole group of the compound of Formula (II) are in a *cis* position, thereby producing a compound of Formula (III) with corresponding hydrogen atoms in a *cis* position.

In particular embodiments, methods of the invention for producing a compound of Formula (I)(a) further comprise producing a compound of Formula (III)(a) by reduction of a compound of Formula (II)(a): 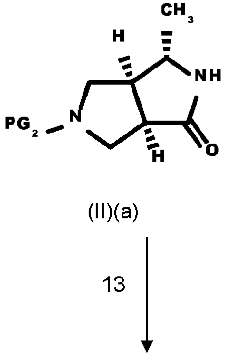 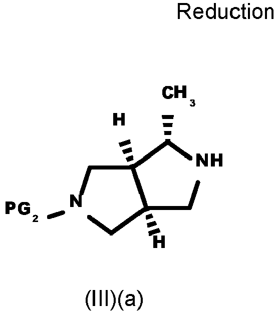 where PG₂ is an amine-protecting group.

In some embodiments, the reduction is carried out using a reducing agent in an aprotic solvent at a temperature from 0°C to reflux.

In particular embodiments, the reducing agent is LiAlH₄, the aprotic solvent is 2-MeTHF, and the reduction is preferably carried out at room temperature.

In some embodiments, methods of the invention for producing a compound of Formula (I) further comprise producing a compound of Formula (II) by deprotection of a compound of formula (XI): 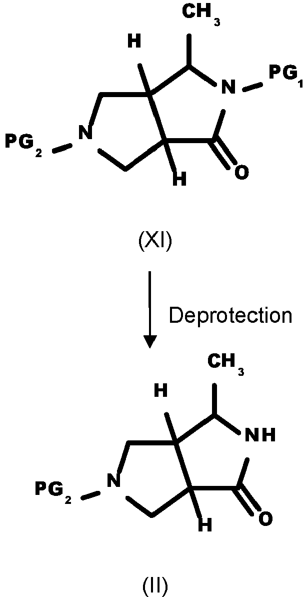 where PG₁ is an amine-protecting group, and PG₂ is an amine protecting group.

In some embodiments, the central hydrogen atoms on the methylated octahydropyrrolo[3,4-c]pyrrole group of the compound of Formula (XI) are in a *cis* position, thereby producing a compound of Formula (II) with corresponding hydrogen atoms in a *cis* position.

In particular embodiments, methods of the invention for producing a compound of Formula (I)(a) further comprise producing a compound of Formula (II)(a) by deprotection of a compound of Formula (XI)(a): 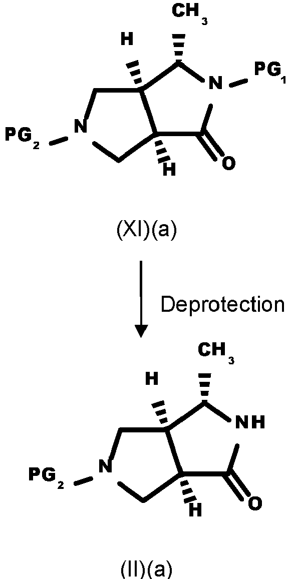 where PG₁ is an amine-protecting group and PG₂ is an amine-protecting group.

In some embodiments, the deprotection is an acid-mediated deprotection in an aprotic solvent.

In particular embodiments, the acid is TFA, and the solvent is DCM, and the deprotection is preferably carried out at a temperature from 0°C to room temperature.

In some embodiments, methods of the invention for producing a compound of Formula (I) further comprise producing a compound of Formula (XI) from a compound of Formula (X) by a cyclisation reaction with a compound of Formula (XVII): 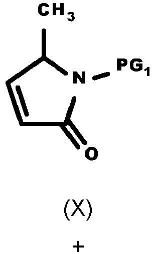 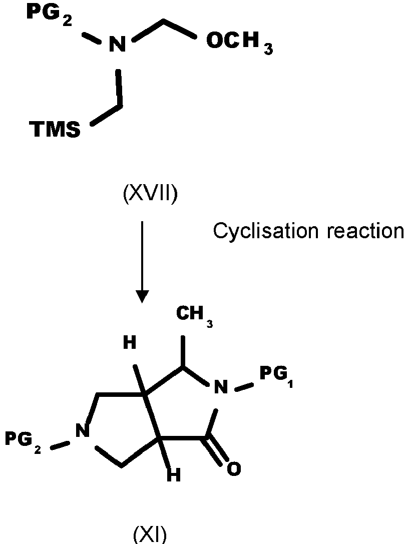 where PG₁ is an amine-protecting group and PG₂ is an amine-protecting group.

It will be appreciated that the choice of reagent(s) for use in the cyclisation will depend on which stereoisomer, or mixture of stereoisomers, is to be produced.

In some embodiments, the cyclisation reaction is selected such that the central hydrogen atoms on the methylated octahydropyrrolo[3,4-c]pyrrole group of the compound of Formula (XI) are in a *cis* position.

In particular embodiments, methods of the invention for producing a compound of Formula (I)(a) further comprise producing a compound of Formula (XI)(a) from a compound of Formula (X)(a) by an acid-mediated cyclisation reaction with a compound of Formula (XVII): 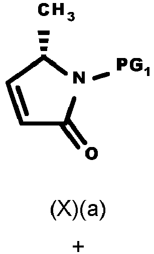 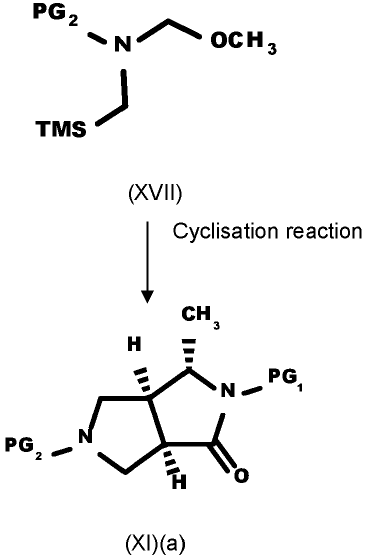 where PG₁ is an amine-protecting group and PG₂ is an amine-protecting group.

In some embodiments, the acid-mediated cyclisation reaction is carried out in the presence of an acid in an aprotic solvent.

In particular embodiments, the acid is TFA, the aprotic solvent is DCM, and the reaction is preferably carried out at a temperature from 0°C to room temperature.

In some embodiments, methods of the invention for producing a compound of Formula (I)(a) further comprise preparing a compound of Formula (X)(a) from a compound of Formula (VI)(a), (VII)(a), (VIII)(a), and (IX)(a), as illustrated by **Scheme 2** below. 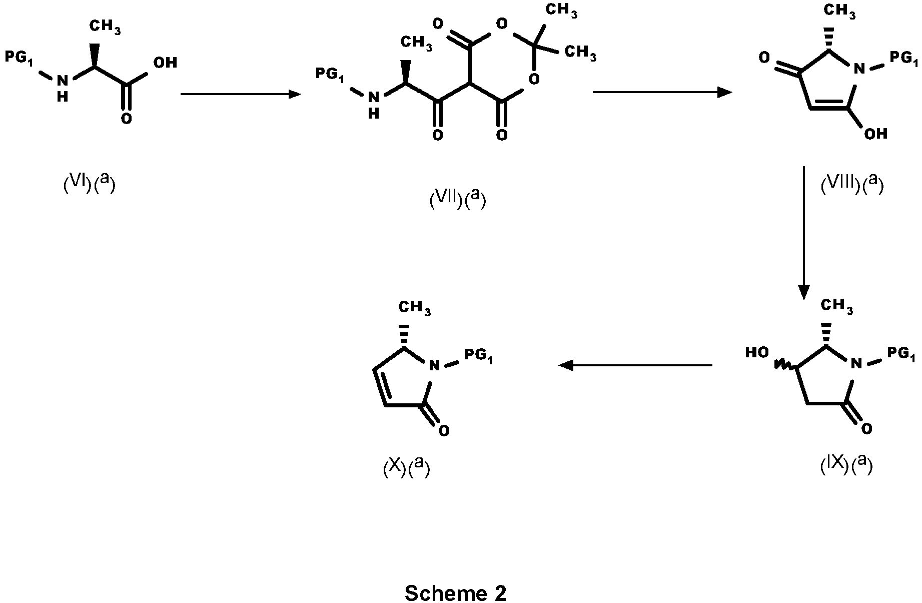 where PG₁ is an amine protecting group.

Alternatively, in other embodiments, methods of the invention for producing a compound of Formula (I)(a) further comprise preparing a compound of Formula (X)(a) from a compound of Formula (XII)(a), (XIII)(a), (XIV)(a), and (XV)(a) as illustrated by **Scheme 3** below. 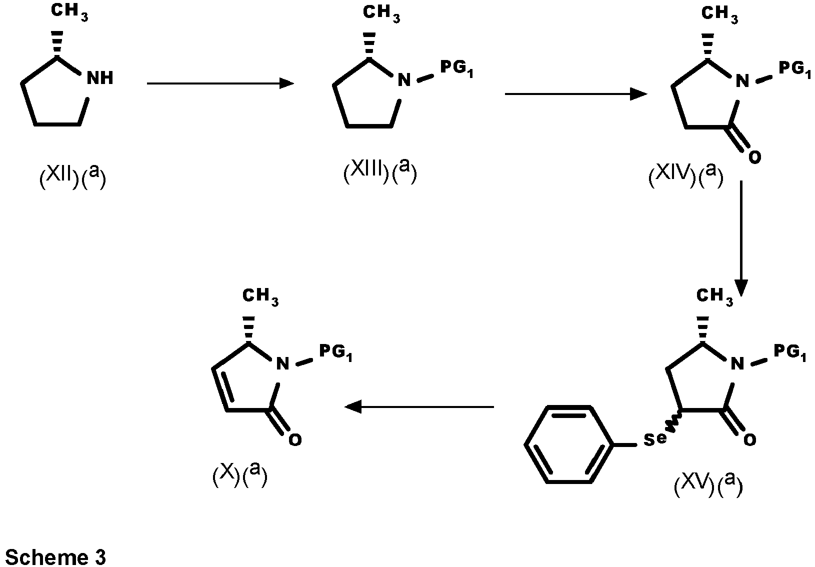 where PG₁ is an amine protecting group.

A compound of Formula (I)(a) may alternatively be prepared from a compound of Formula (III)(a), (XVIII)(a), (XIX)(a), (XVI), (XX)(a), and (XXI)(a) as illustrated by **Scheme 4** below. 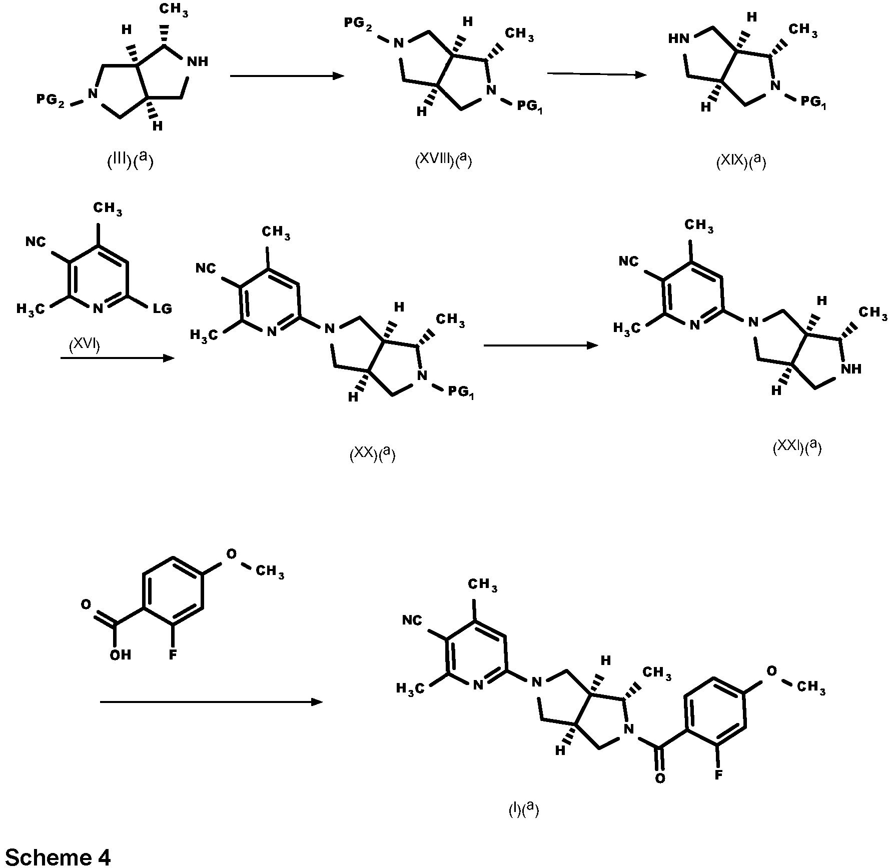 where PG₁ is an amine protecting group, and where PG₂ is an amine protecting group. LG is a leaving group.

The invention also provides a method of producing a compound of Formula (I), which comprises reacting a compound of Formula (XXI) and 2-fluoro-4-methoxybenzoic acid to produce the compound of Formula (I):

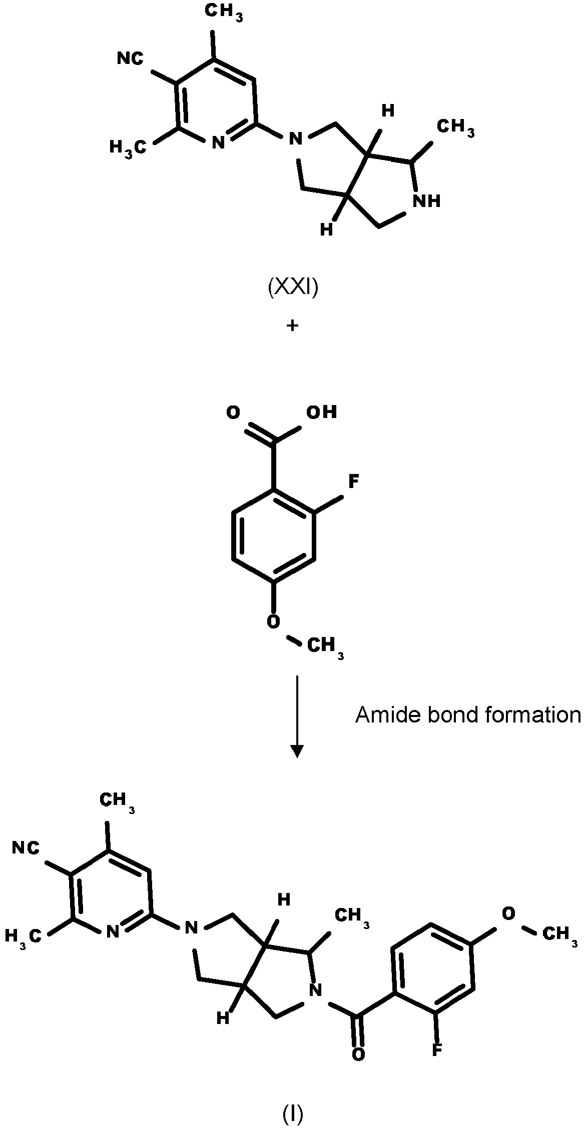

In some embodiments, the central hydrogen atoms on the methylated octahydropyrrolo[3,4-c]pyrrole group of the compound of Formula (XXI) are in a *cis* position, thereby producing a compound of Formula (I) with corresponding hydrogen atoms in a *cis* position.

In particular embodiments, there is provided a method of producing a compound of Formula (I)(a), which comprises reacting a compound of Formula (XXI)(a) and 2-fluoro-4-methoxybenzoic acid to produce the compound of Formula (I)(a):

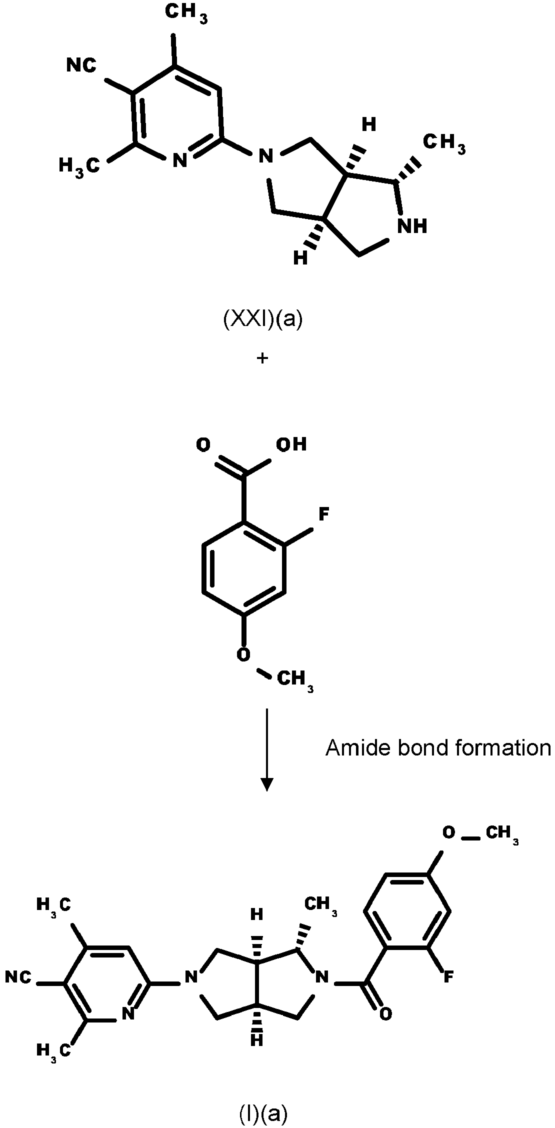

In some embodiments, the compound of Formula (XXI) or (XXI)(a) and 2-fluoro-4-methoxybenzoic acid are reacted in the presence of a coupling reagent and an organic base, in an aprotic solvent, at a temperature from 0-100°C.

In particular embodiments, the coupling reagent is (HATU, orT3P, the organic base is DIPEA or Et₃N, the aprotic solvent is DMF or MeCN, and the temperature is preferably room temperature.

A skilled person will appreciate that alternative coupling conditions are available in combination with a range of alternative bases and solvents.

In some embodiments, methods of the invention for producing a compound of Formula (I) further comprise producing a compound of Formula (XXI) by deprotection of a compound of Formula (XX): 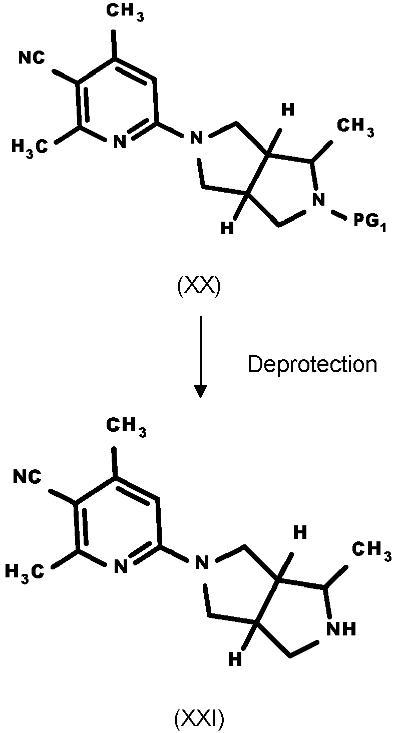 where PG₁ is an amine-protecting group.

In some embodiments, the central hydrogen atoms on the methylated octahydropyrrolo[3,4-c]pyrrole group of the compound of Formula (XX) are in a *cis* position, thereby producing a compound of Formula (XXI) with corresponding hydrogen atoms in a *cis* position.

In particular embodiments, methods of the invention for producing a compound of Formula (I)(a) further comprise producing a compound of Formula (XXI)(a) by deprotection of a compound of Formula (XX)(a): 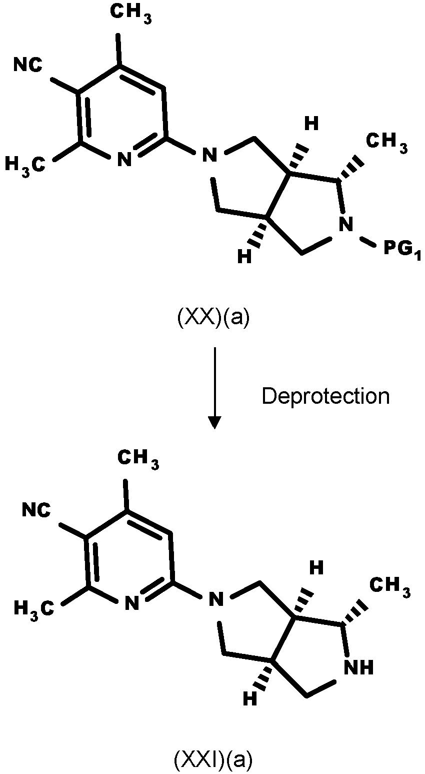 where PG₁ is an amine-protecting group.

In some embodiments, the deprotection is an acid-mediated deprotection in an aprotic solvent.

In particular embodiments (in particular where PG₁ = ^{t}BoC), the acid is TFA, and the solvent is DCM, and the deprotection is preferably carried out at a temperature from 0°C to room temperature.

In some embodiments, methods of the invention for producing a compound of Formula (I) further comprise producing a compound of Formula (XX) by reacting a compound of Formula (XIX) and a compound of Formula (XVI) to produce the compound of Formula (XX): 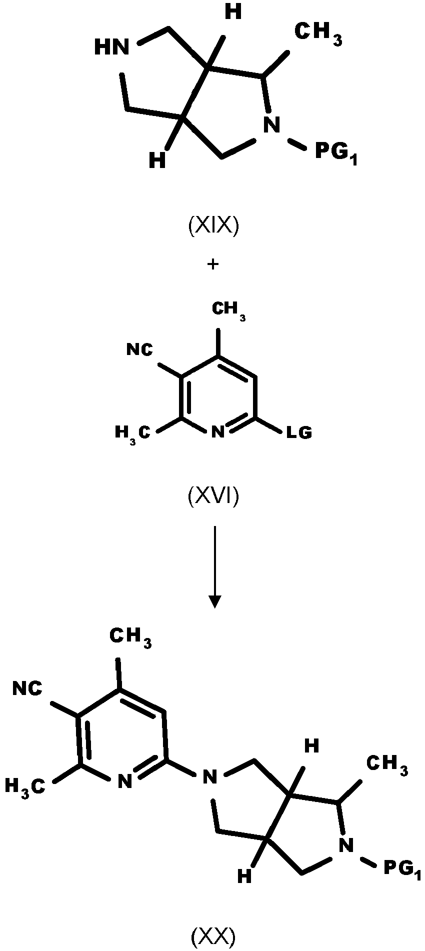 where PG₁ is an amine-protecting group, and LG is a leaving group.

Examples of suitable leaving groups include: a chloro, bromo, mesylate, or tosylate group. In particular embodiments, LG is chloro.

In some embodiments, the central hydrogen atoms on the methylated octahydropyrrolo[3,4-c]pyrrole group of the compound of Formula (XIX) are in a *cis* position, thereby producing a compound of Formula (XX) with corresponding hydrogen atoms in a *cis* position.

In particular embodiments, methods of the invention for producing a compound of Formula (I)(a) further comprise producing a compound of Formula (XX)(a) by reacting a compound of Formula (XIX)(a) and a compound of Formula (XVI) to produce the compound of Formula (XX)(a): 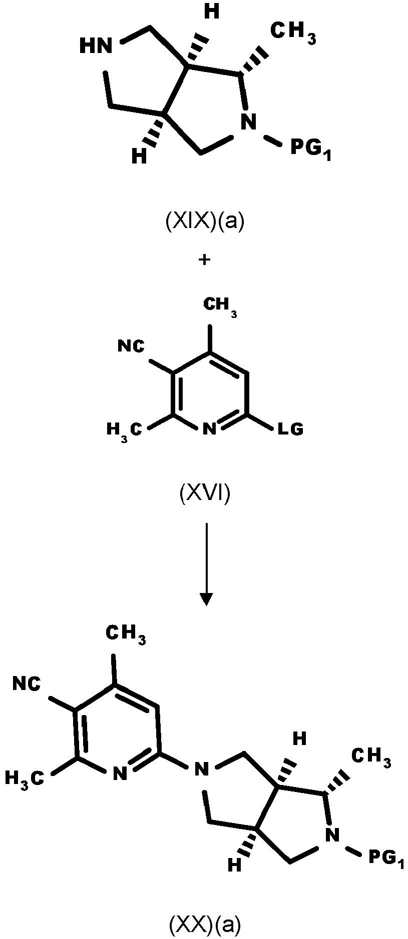 where PG₁ is an amine-protecting group, and LG is a leaving group.

Typically, the compound of Formula (XIX) or (XIX)(a) is reacted with the compound of Formula (XVI) in the presence of a base in a solvent.

In some embodiments, the compound of Formula (XIX) or (XIX)(a) is reacted with the compound of Formula (XVI) in the presence of a base in a solvent. In particular embodiments, the base is Cs₂CO₃ or K₂CO₃, and the solvent is DMF or EtOH, and the reaction is carried out at an elevated temperature, such as 80-110°C.

In some embodiments, methods of the invention for producing a compound of Formula (I) further comprise producing a compound of Formula (XIX) by deprotection of a compound of Formula (XVIII) to produce the compound of Formula (XIX): 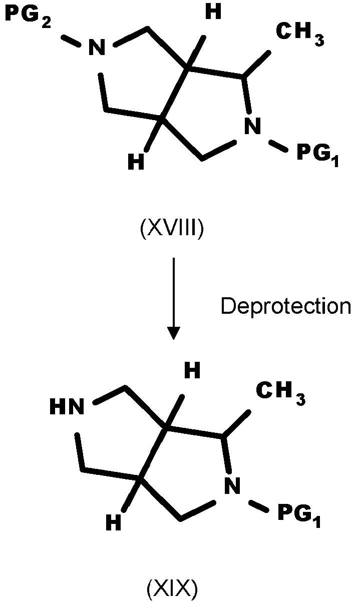 where PG₁ is an amine-protecting group, and PG₂ is an amine-protecting group.

In some embodiments, the central hydrogen atoms on the methylated octahydropyrrolo[3,4-c]pyrrole group of the compound of Formula (XVIII) are in a *cis* position, thereby producing a compound of Formula (XIX) with corresponding hydrogen atoms in a *cis* position.

In particular embodiments, methods of the invention for producing a compound of Formula (I)(a) further comprise producing a compound of Formula (XIX)(a) by deprotection of a compound of Formula (XVIII)(a) to produce the compound of Formula (XIX)(a): 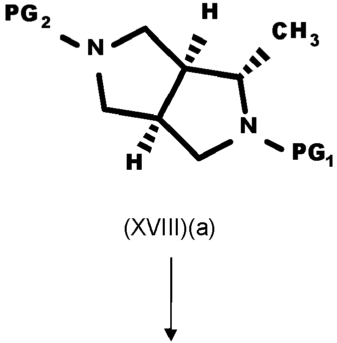 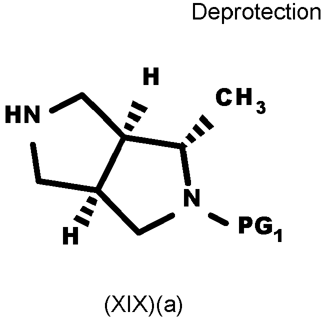 where PG₁ is an amine-protecting group, and PG₂ is an amine-protecting group.

In some embodiments (in particular where PG₂ = Bn), the deprotection is carried out by transfer palladium catalysed hydrogenation. For example, transfer hydrogenation may be carried out using ammonium formate and Pd(OH)₂ on charcoal, in ethanol, at a temperature between room temperature and reflux.

A skilled person will appreciate that alternative hydrogenation conditions are available to effect this transformation.

In some embodiments, methods of the invention for producing a compound of Formula (I) further comprise producing a compound of Formula (XVIII) by protecting a compound of Formula (III) to produce the compound of Formula (XVIII): 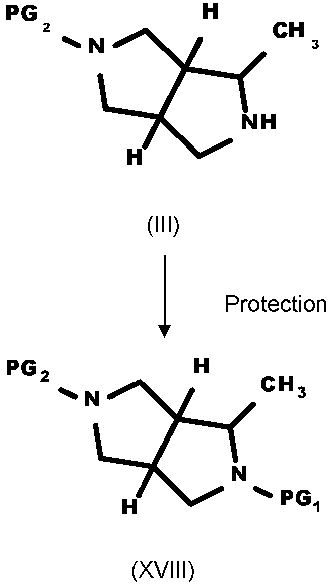 where PG₁ is an amine-protecting group, and PG₂ is an amine-protecting group.

In some embodiments, the central hydrogen atoms on the methylated octahydropyrrolo[3,4-c]pyrrole group of the compound of Formula (III) are in a *cis* position, thereby producing a compound of Formula (XVIII) with corresponding hydrogen atoms in a *cis* position.

In particular embodiments, methods of the invention for producing a compound of Formula (I)(a) further comprise producing a compound of Formula (XVIII)(a) by protecting a compound of Formula (III)(a) to produce the compound of Formula (XVIII)(a): 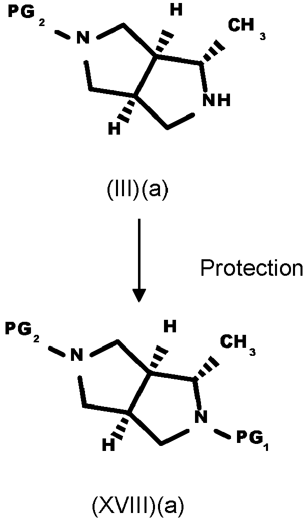 where PG₁ is an amine-protecting group, and PG₂ is an amine-protecting group.

In particular embodiments, protection is carried out under standard conditions for introducing an amine protecting group. In some embodiments (in particular where PG₁ = ^{t}BoC), protection is carried out by treatment with BOC₂O in a suitable solvent, such as DCM, typically at room temperature.

In a further aspect, there is provided according to the invention a method of producing a compound of Formula (XI), which comprises cyclisation of a compound of Formula (X) with a compound of Formula (XVII) to produce a compound of Formula (XI): 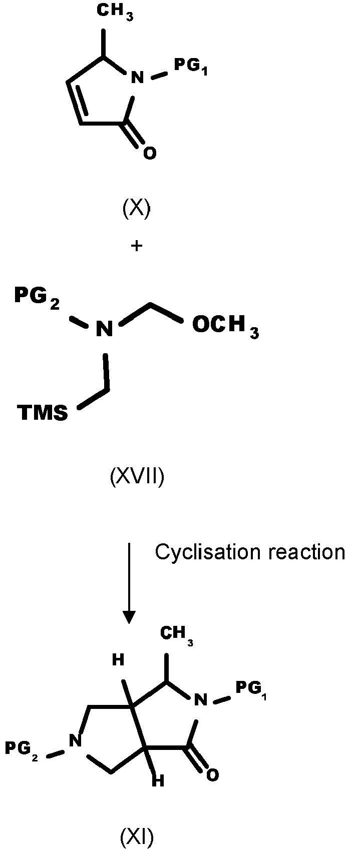 where PG₁ is an amine-protecting group, and PG₂ is an amine-protecting group.

It will be appreciated that the choice of reagent(s) for use in the cyclisation will depend on which stereoisomer, or mixture of stereoisomers, is to be produced.

In some embodiments, the cyclisation reaction is selected such that the central hydrogen atoms on the methylated octahydropyrrolo[3,4-c]pyrrole group of the compound of Formula (XI) are in a *cis* position.

In particular embodiments, there is provided a method of producing a compound of Formula (XI)(a), which comprises acid-mediated cyclisation of a compound of Formula (X)(a) with a compound of Formula (XVII) to produce a compound of Formula (XI)(a): 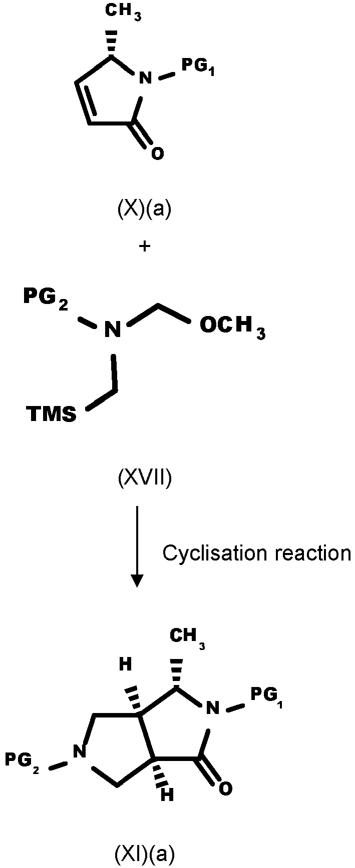 where PG₁ is an amine-protecting group, and PG₂ is an amine-protecting group.

In some embodiments, a method of the invention for producing a compound of Formula (XI) further comprises converting the compound of Formula (XI) to a compound of Formula (I).

In particular embodiments, a method of the invention for producing a compound of Formula (XI)(a) further comprises converting the compound of Formula (XI)(a) to a compound of Formula (I)(a).

There is also provided according to the invention use of a compound of Formula (X) in a method of producing a compound of Formula (I).

There is also provided according to the invention use of a compound of Formula (X)(a) in a method of producing a compound of Formula (I)(a).

In some embodiments, methods of the invention for producing a compound of Formula (I) further comprise reacting the compound of Formula (I) to yield a corresponding pharmaceutically acceptable salt of the compound of Formula (I).

In some embodiments, methods of the invention for producing a compound of Formula (I)(a) further comprise reacting the compound of Formula (I)(a) to yield a corresponding pharmaceutically acceptable salt of the compound of Formula (I)(a).

There is also provided according to the invention, a compound of Formula (II), (II)(a), (III), (III)(a), (IV), (IV)(a), (V), (V)(a), (VI), (VI)(a), (VII), (VII)(a), (VIII), (VIII)(a), (IX), (IX)(a), (X), (X)(a), (XI), (XI)(a), (XII), (XII)(a), (XIII), (XIII)(a), (XIV), (XIV)(a), (XV), (XV)(a), (XVI), (XVII), (XVIII), (XVIII)(a), (XIX), (XIX)(a), (XX), (XX)(a), (XXI), or (XXI)(a), or a pharmaceutically acceptable salt, solvate, hydrate, geometrical isomer, tautomer, or optical isomer thereof.

Examples of suitable amine protecting groups are well-known to the skilled person, and include those described in 'Greene's Protective Groups in Organic Synthesis' by Theodora W Greene and Peter G M Wuts, fifth edition, (John Wiley and Sons, 2014), in particular Chapter 7 ("Protection for the Amino Group")*,* incorporated herein by reference, which also describes methods for the removal of such groups.

Particular examples of suitable amine protecting groups include the following:
Carbobenzyloxy (Cbz) group - removed by hydrogenolysis;
*p*-Methoxybenzyl carbonyl (Moz or MeOZ) group - removed by hydrogenolysis, more labile than Cbz;
*tert*-Butyloxycarbonyl (^{t}BOC) group (common in solid phase peptide synthesis) - removed by concentrated strong acid (such as HCI or CF₃COOH), or by heating to >80 °C;
9-Fluorenylmethyloxycarbonyl (FMOC) group - removed by base, such as piperidine;
Acetyl (Ac) group - removed by treatment with a base, most often, with aqueous or gaseous ammonia or methylamine;
Benzoyl (Bz) group - removed by treatment with a base, most often with aqueous or gaseous ammonia or methylamine;
Benzyl (Bn) group - removed by hydrogenolysis;
Carbamate group - removed by acid and mild heating, for example *tert*-butyl carbamate;
*p*-Methoxybenzyl (PMB) - removed by hydrogenolysis, more labile than benzyl;
3,4-Dimethoxybenzyl (DMPM) - removed by hydrogenolysis, more labile than *p*-methoxybenzyl; *p*-methoxyphenyl (PMP) group - removed by ammonium cerium(IV) nitrate (CAN);
Tosyl (Ts) group - removed by concentrated acid (HBr, H₂SO4) and strong reducing agents (sodium in liquid ammonia or sodium naphthalenide);
Trichloroethyl chloroformate (Troc) group - removed by Zn insertion in the presence of acetic acid;
Other Sulfonamides (Nosyl and Nps) groups - removed by samarium iodide, tributyltin hydride; Acetamide;
Benzamide;
9-Fluorenylmethyl carbamate;
Benzhydryl;
Trityl.

Thus, PG₁ and PG₂ may each independently be selected from: a Cbz, MeOZ, ^{t}BOC, FMOC, Ac, Bz, Bn, carbamate, PMB, Ts, Troc, sulphonamide, acetamide, benzamide, *tert*-butylcarbamate, 9-Fluorenylmethyl carbamate, benzhydryl, trityl group.

In particular embodiments, PG₁ is a carbamate group, preferably *t*-butyl carbamate

In particular embodiments PG₂ is benzyl.

In particular embodiments, PG₁ is a carbamate group, preferably *t*-butyl carbamate, and PG₂ is benzyl.

Examples of suitable leaving groups include: a chloro, bromo, mesylate, or tosylate group.

In particular embodiments, LG is chloro.

Examples of methods of producing a compound of Formula (I), or (I)(a), in accordance with the invention are described in detail below.

The compounds of the invention include compounds of formula (I), or (I)(a), and salts thereof as hereinafter defined, polymorphs, and isomers thereof (including optical, geometric and tautomeric isomers) as hereinafter defined and isotopically-labelled compounds of formula (I) or (I)(a).

Any formula given herein is intended to represent compounds having structures depicted by the structural formula as well as certain variations or forms. In particular, compounds of any formula given herein may have asymmetric centres and therefore exist in different enantiomeric forms. All optical isomers and stereoisomers of the compounds of the general formula, and mixtures thereof, are considered within the scope of the formula. Thus, any formula given herein is intended to represent a racemate, one or more enantiomeric forms, one or more diastereomeric forms, one or more atropisomeric forms, and mixtures thereof. Furthermore, certain structures may exist as geometric isomers (i.e., *cis* and *trans* isomers), as tautomers, or as atropisomers. Where a compound of Formula (I), or (I)(a), contains for example, a keto or guanidine group or an aromatic moiety, tautomeric isomerism ('tautomerism') can occur. It follows that a single compound may exhibit more than one type of isomerism.

Included within the scope of the claimed compounds of the present invention are all stereoisomers, geometric isomers and tautomeric forms of the compounds of Formula (I), or (I)(a), including compounds exhibiting more than one type of isomerism, and mixtures of one or more thereof. Also included are acid addition or base addition salts wherein the counter ion is optically active, for example, D-lactate or L-lysine, or racemic, for example, DL-tartrate or DL-arginine.

Examples of types of potential tautomerisms shown by the compounds of the invention include; amide ⇔ hydroxyl-imine and keto ⇔ enol tautomersims:

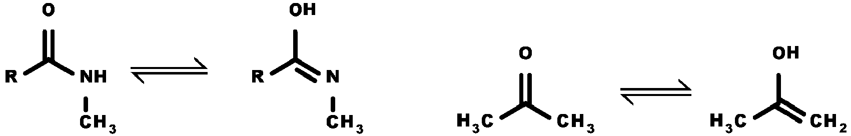

*Cis*/*trans* isomers may be separated by conventional techniques well known to those skilled in the art, for example, by chromatography and fractional crystallisation.

Conventional techniques for the preparation/isolation of individual enantiomers include chiral synthesis from a suitable optically pure precursor or resolution of the racemate (or the racemate of a salt or other derivative) using, for example, chiral high pressure liquid chromatography (HPLC).

Chiral compounds of the invention (and chiral precursors thereof) may be obtained in enantiomerically-enriched form using chromatography, typically HPLC, on a resin with an asymmetric stationary phase and with a mobile phase consisting of a hydrocarbon, typically heptane or hexane, containing from 0 to 50% ethanol, typically from 2 to 20%. Concentration of the eluate affords the enriched mixture.

Mixtures of stereoisomers may be separated by conventional techniques known to those skilled in the art (see, for example, *"*Stereochemistry of Organic Compounds" by E L Eliel (Wiley, New York, 1994)).

Additionally, any formula given herein is intended to refer also to hydrates, solvates, and polymorphs of such compounds, and mixtures thereof, even if such forms are not listed explicitly. A compound of Formula (I), or (I)(a), or pharmaceutically acceptable salt of a compound of Formula (I), or (I)(a), may be obtained as a solvate. Solvates include those formed from the interaction or complexation of compounds of the invention with one or more solvents, either in solution or as a solid or crystalline form. In some embodiments, the solvent is water and then the solvates are hydrates. In addition, certain crystalline forms of a compound of Formula (I), or (I)(a), or a pharmaceutically acceptable salt of a compound of Formula (I), or (I)(a), may be obtained as co-crystals. In certain embodiments of the invention, a compound of Formula (I), or (I)(a), or a pharmaceutically acceptable salt of a compound of Formula (I), or (I)(a), may be obtained in a crystalline form. In other embodiments, a compound of Formula (I), or (I)(a), may be obtained in one of several polymorphic forms, as a mixture of crystalline forms, as a polymorphic form, or as an amorphous form. In other embodiments, a compound of Formula (I), or (I)(a), may convert in solution between one or more crystalline forms and/or polymorphic forms.

Any formula given herein is also intended to represent unlabelled forms as well as isotopically labelled forms of the compounds. Isotopically labelled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, and fluorine, such as ²H, ³H, ¹¹C, ¹³C, ¹⁴C, ¹³N, ¹⁵N, ¹⁵O, ¹⁷0, ¹⁸0, ¹⁸F, respectively. Such isotopically labelled compounds are useful in metabolic studies (preferably with ¹⁴C), reaction kinetic studies (with, for example ²H or ³H), detection or imaging techniques (such as positron emission tomography (PET) or single-photon emission computed tomography (SPECT)) including drug or substrate tissue distribution assays, or in radioactive treatment of subjects. Substitution with positron emitting isotopes, such as ¹¹C, ¹⁸F, ¹⁵O and ¹³N, can be useful in PET studies for examining substrate receptor occupancy. In particular, an ¹⁸F or ¹¹C labelled compound may be particularly preferred for PET studies. Further, substitution with heavier isotopes such as deuterium (i.e., ²H) may afford certain therapeutic advantages resulting from greater metabolic stability, for example increased *in vivo* half-life or reduced dosage requirements. Certain isotopically-labelled compounds of formula (I) for example, those incorporating a radioactive isotope, are useful in drug and/or substrate tissue distribution studies. The radioactive isotopes tritium, *i.e*. ³H, and carbon-14, *i.e*. ¹⁴C, are particularly useful for this purpose in view of their ease of incorporation and ready means of detection.

Isotopically labelled compounds of this invention and prodrugs thereof can generally be prepared by carrying out the procedures disclosed in the schemes or in the examples and preparations described below by substituting a readily available isotopically labelled reagent for a non-isotopically labelled reagent.

A compound of Formula (I), or (I)(a), and pharmaceutically acceptable salts thereof, may be used alone or in combination with one or more additional active ingredients to formulate pharmaceutical compositions. A pharmaceutical composition therefore comprises an effective amount of a compound of Formula (I), or (I)(a), or a pharmaceutically acceptable salt thereof. The invention includes also pharmaceutically acceptable salts of a compound of Formula (I) or (I)(a). A "pharmaceutically acceptable salt" is intended to mean a salt of a free acid or base of a compound represented by Formula (I), or (I)(a), that is non-toxic, biologically tolerable, or otherwise biologically suitable for administration to a subject. See, generally, G.S. Paulekuhn, et al., "Trends in Active Pharmaceutical Ingredient Salt Selection based on Analysis of the Orange Book Database", J. Med. Chem., 2007, 50:6665-72, S.M. Berge, et al., "Pharmaceutical Salts", J Pharm Sci., 1977, 66:1 -19, and Handbook of Pharmaceutical Salts, Properties, Selection, and Use, Stahl and Wermuth, Eds., Wiley-VCH and VHCA, Zurich, 2002.

Examples of pharmaceutically acceptable salts are those that are pharmacologically effective and suitable for contact with the tissues of subjects without undue toxicity, irritation, or allergic response. A compound of Formula (I), or (I)(a), may possess a sufficiently acidic group, a sufficiently basic group, or both types of functional groups, and accordingly react with a number of inorganic or organic bases, and inorganic and organic acids, to form a pharmaceutically acceptable salt. bases, and inorganic and organic acids, to form a pharmaceutically acceptable salt.

Examples of pharmaceutically acceptable salts include sulfates, pyrosulfates, bisulfates, sulfites, bisulfites, phosphates, monohydrogen- phosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1 ,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycolates, tartrates, methane-sulfonates, propanesulfonates, naphthalene-1 -sulfonates, naphthalene-2-sulfonates, and mandelates.

The disclosure also relates to pharmaceutically acceptable prodrugs of a compound of Formula (I) or (I)(a), and treatment methods employing such pharmaceutically acceptable prodrugs. The term "prodrug" means a precursor of a designated compound that, following administration to a subject, yields the compound *in vivo* via a chemical or physiological process such as solvolysis or enzymatic cleavage, or under physiological conditions (e.g., a prodrug on being brought to physiological pH is converted to the compound of Formula (I), or (I)(a)). A "pharmaceutically acceptable prodrug" is a prodrug that is non-toxic, biologically tolerable, and otherwise biologically suitable for administration to the subject. Illustrative procedures for the selection and preparation of suitable prodrug derivatives are described, for example, in *"*Design of Prodrugs", ed. H. Bundgaard, Elsevier, 1985.

The present disclosure also relates to pharmaceutically active metabolites of a compound of Formula (I), or (I)(a), which may also be used in the methods of the invention. A "pharmaceutically active metabolite" means a pharmacologically active product of metabolism in the body of a compound of Formula (I), or (I)(a), or salt thereof. Prodrugs and active metabolites of a compound may be determined using routine techniques known or available in the art. See, e.g., Bertolini, et al., J Med Chem. 1997, 40, 201 1 -2016; Shan, et al., J Pharm Sci. 1997, 86 (7), 765-767; Bagshawe, Drug Dev Res. 1995, 34, 220-230; Bodor, Adv Drug Res. 1984, 13, 224-331 ; Bundgaard, Design of Prodrugs (Elsevier Press, 1985); and Larsen, Design and Application of Prodrugs, Drug Design and Development (Krogsgaard-Larsen, et al., eds., Harwood Academic Publishers, 1991).

A compound of Formula (I), or (I)(a), and its pharmaceutically acceptable salts, pharmaceutically acceptable prodrugs, and pharmaceutically active metabolites, are useful as antagonists of the orexin receptor.

The term "treat", "treating", or "treatment" as used herein is intended to refer to administration of a compound or composition of the invention to a subject for the purpose of effecting a therapeutic or prophylactic benefit through modulation (in particular, antagonism) of orexin receptor activity. Treating includes reversing, ameliorating, alleviating, inhibiting the progress of, lessening the severity of, or preventing a disease, disorder, or condition, or one or more symptoms of such disease, disorder or condition mediated through modulation of orexin receptor activity. The term "subject" refers to a mammalian patient in need of such treatment, such as a human.

Accordingly, the invention relates to compounds or compositions described herein for use in treating subjects diagnosed with or suffering from a disease, disorder, or condition mediated by orexin receptor activity, such as: disorders of the sleep-wake cycle, metabolic disorders, neurological disorders, and other disorders (e.g., feeding, drinking, arousal, stress, addiction, especially drug addiction, metabolism and reproduction). Symptoms or disease states are intended to be included within the scope of "conditions, disorders, or diseases."

Sleep disorders include, but are not limited to, disorders of the sleep-wake cycle, sleep-wake transition disorders, insomnia, restless legs syndrome, jet-lag, disturbed sleep, and sleep disorders secondary to neurological disorders (e.g., manias, depressions, manic depression, schizophrenia, and pain syndromes (e.g., fibromyalgia, neuropathic).

Metabolic disorders include, but are not limited to, overweight or obesity and conditions related to overweight or obesity, such as insulin resistance, type II diabetes, hyperlipidemia, gallstones, angina, hypertension, breathlessness, tachycardia, arrhythmias, angina pectoris, acute heart failure, infertility, sleep apnoea, back and joint pain, varicose veins and osteoarthritis. Neurological disorders include, but are not limited to, Parkinson's disease, Alzheimer's disease, Huntington's disease, Tourette's Syndrome, catatonia, anxiety, stress disorder, panic disorder, delirium and dementias.

Other disorders include, but are not limited to, ulcers, irritable bowel syndrome, diarrhoea and gastroesophageal reflux, addiction and alcoholism.

It will be appreciated that compounds of the invention are particularly advantageous for treatment of various diseases (such as, but not restricted to, sleep disorders) because they exhibit rapid and high levels of intestinal absorption, leading to rapid achievement of maximal concentration in the body and the brain, and a sufficient but relatively short exposure period.

In treatment methods according to the invention, an effective amount of a compound of the invention is administered to a subject suffering from or diagnosed as having such a disease, disorder, or condition. An "effective amount" means an amount or dose sufficient to generally bring about the desired therapeutic or prophylactic benefit in a subject in need of such treatment for the designated disease, disorder, or condition. Effective amounts or doses of a compound of the present invention may be ascertained by routine methods such as modelling, dose escalation studies or clinical trials, and by taking into consideration routine factors, e.g., the mode or route of administration or drug delivery, the pharmacokinetics of the compound, the severity and course of the disease, disorder, or condition, the subject's previous or ongoing therapy, the subject's health status and response to drugs, and the judgment of the treating physician. An example of a dose is in the range of from about 0.001 to about 200 mg of compound per kg of subject's body weight per day, preferably about 0.05 to 100 mg/kg/day, or about 1 to 35 mg/kg/day, in single or divided dosage units (e.g., BID, TID, QID). For a 70kg human, an illustrative range for a suitable dosage amount is from about 0.05 to about 7 g/day, or about 0.2 to about 2.5 g/day.

Once improvement of the subject's disease, disorder, or condition has occurred, the dose may be adjusted for preventative or maintenance treatment. For example, the dosage or the frequency of administration, or both, may be reduced as a function of the symptoms, to a level at which the desired therapeutic or prophylactic effect is maintained. Of course, if symptoms have been alleviated to an appropriate level, treatment may cease. Subjects may, however, require intermittent treatment on a long-term basis upon any recurrence of symptoms.

In addition, the active agents of the invention may be used in combination with additional active ingredients in the treatment of the above conditions. The additional active ingredients may be co-administered separately with an active agent of the invention, or included with such an agent in a pharmaceutical composition according to the invention. In an exemplary embodiment, additional active ingredients are those that are known or discovered to be effective in the treatment of conditions, disorders, or diseases mediated by orexin receptor activity, such as another orexin modulator or a compound active against another target associated with the particular condition, disorder, or disease. The combination may serve to increase efficacy (e.g., by including in the combination a compound potentiating the potency or effectiveness of an active agent according to the invention), decrease one or more side effects, or decrease the required dose of the active agent according to the invention.

The active agents of the invention are used, alone or in combination with one or more additional active ingredients, to formulate pharmaceutical compositions of the invention. A pharmaceutical composition of the invention comprises: (a) an effective amount of at least one active agent in accordance with the invention; and (b) a pharmaceutically acceptable excipient.

A "pharmaceutically acceptable excipient" refers to a substance that is non-toxic, biologically tolerable, and otherwise biologically suitable for administration to a subject, such as an inert substance, added to a pharmacological composition or otherwise used as a vehicle, carrier, or diluent to facilitate administration of an agent and that is compatible therewith. Examples of excipients include calcium carbonate, calcium phosphate, various sugars and types of starch, cellulose derivatives, gelatin, vegetable oils, and polyethylene glycols.

Delivery forms of the pharmaceutical compositions containing one or more dosage units of the active agents may be prepared using suitable pharmaceutical excipients and compounding techniques known or that become available to those skilled in the art. The compositions may be administered in the inventive methods by a suitable route of delivery, e.g., oral, parenteral, rectal, topical, or ocular routes, or by inhalation.

The preparation may be in the form of tablets, capsules, sachets, dragees, powders, granules, lozenges, powders for reconstitution, liquid preparations, or suppositories. Preferably, the compositions are formulated for intravenous infusion, topical administration, or oral administration.

For oral administration, the compounds of the invention can be provided in the form of tablets or capsules, or as a solution, emulsion, or suspension. To prepare the oral compositions, the compounds may be formulated to yield a dosage of, e.g., from about 0.05 to about 100 mg/kg daily, or from about 0.05 to about 35 mg/kg daily, or from about 0.1 to about 10 mg/kg daily. For example, a total daily dosage of about 5 mg to 5 g daily may be accomplished by dosing once, twice, three, or four times per day.

Oral tablets may include a compound according to the invention mixed with pharmaceutically acceptable excipients such as inert diluents, disintegrating agents, binding agents, lubricating agents, sweetening agents, flavouring agents, colouring agents and preservative agents. Suitable inert fillers include sodium and calcium carbonate, sodium and calcium phosphate, lactose, starch, sugar, glucose, methyl cellulose, magnesium stearate, mannitol, sorbitol, and the like. Exemplary liquid oral excipients include ethanol, glycerol, water, and the like. Starch, polyvinyl-pyrrolidone (PVP), sodium starch glycolate, microcrystalline cellulose, and alginic acid are suitable disintegrating agents. Binding agents may include starch and gelatin. The lubricating agent, if present, may be magnesium stearate, stearic acid or talc. If desired, the tablets may be coated with a material such as glyceryl monostearate or glyceryl distearate to delay absorption in the gastrointestinal tract, or may be coated with an enteric coating.

Capsules for oral administration include hard and soft gelatin capsules. To prepare hard gelatin capsules, compounds of the invention may be mixed with a solid, semi-solid, or liquid diluent. Soft gelatin capsules may be prepared by mixing the compound of the invention with water, an oil such as peanut oil or olive oil, liquid paraffin, a mixture of mono and di-glycerides of short chain fatty acids, polyethylene glycol 400, or propylene glycol.

Liquids for oral administration may be in the form of suspensions, solutions, emulsions or syrups or may be lyophilized or presented as a dry product for reconstitution with water or other suitable vehicle before use. Such liquid compositions may optionally contain: pharmaceutically-acceptable excipients such as suspending agents (for example, sorbitol, methyl cellulose, sodium alginate, gelatin, hydroxyethylcellulose, carboxymethylcellulose, aluminium stearate gel and the like); non-aqueous vehicles, e.g., oil (for example, almond oil or fractionated coconut oil), propylene glycol, ethyl alcohol, or water; preservatives (for example, methyl or propyl p-hydroxybenzoate or sorbic acid); wetting agents such as lecithin; and, if desired, flavouring or colouring agents.

The active agents of this invention may also be administered by non-oral routes. For example, the compositions may be formulated for rectal administration as a suppository. For parenteral use, including intravenous, intramuscular, intraperitoneal, or subcutaneous routes, the compounds of the invention may be provided in sterile aqueous solutions or suspensions, buffered to an appropriate pH and isotonicity or in parenterally acceptable oil. Suitable aqueous vehicles include Ringer's solution and isotonic sodium chloride. Such forms will be presented in unit-dose form such as ampules or disposable injection devices, in multi-dose forms such as vials from which the appropriate dose may be withdrawn, or in a solid form or pre-concentrate that can be used to prepare an injectable formulation. Illustrative infusion doses may range from about 1 to 1000 µg/kg/minute of compound, admixed with a pharmaceutical carrier over a period ranging from several minutes to several days.

For topical administration, the compounds may be mixed with a pharmaceutical carrier at a concentration of about 0.1 % to about 10% of drug to vehicle. Another mode of administering the compounds of the invention may utilize a patch formulation to affect transdermal delivery. Compounds of the invention may alternatively be administered in methods of this invention by inhalation, via the nasal or oral routes, e.g., in a spray formulation also containing a suitable carrier.

Exemplary compounds of the invention, and exemplary compounds useful in methods of the invention will now be described by reference to the illustrative synthetic schemes for their general preparation below and the specific examples that follow. Artisans will recognize that, to obtain the various compounds herein, starting materials may be suitably selected so that the ultimately desired substituents will be carried through the reaction scheme with or without protection as appropriate to yield the desired product. Alternatively, it may be necessary or desirable to employ, in the place of the ultimately desired substituent, a suitable group that may be carried through the reaction scheme and replaced as appropriate with the desired substituent. Unless otherwise specified, the variables are as defined above in reference to Formula (I). Reactions may be performed between the melting point and the reflux temperature of the solvent, and preferably between 0 °C and the reflux temperature of the solvent. Reactions may be heated employing conventional heating or microwave heating. Reactions may also be conducted in sealed pressure vessels above the normal reflux temperature of the solvent.

Abbreviations and acronyms used herein include the following:

| **Abbreviation/acronym** | **Term** |
|---|---|
| AcOH | Acetic acid |
| aq | aqueous |
| Bn | benzyl |
| Boc | *tert*-butoxycarbonyl |
| Boc₂O | di-*tert*-butyl dicarbonate |
| br | broad |
| °C | degrees Celsius |
| CDCl₃ | deutero-chloroform |
| CS₂CO₃ | Cesium carbonate |
| Cy | cyclohexane |
| δ | chemical shift |
| d | doublet |
| dd | double doublet |
| ddd | Doublet of doublets of doublets |
| DCM | dichloromethane |
| DIPEA | N-ethyldiisopropylamine or N,N-diisopropylethylamine |
| DMAP | 4-(dimethylamino)pyridine |
| DMF | N, N-dimethylformamide |
| DMMN | Dimethylmalonitrile |
| DMSO | Dimethyl sulfoxide |
| DMSO-d₆ | hexadeuterodimethyl sulfoxide |
| EDC.HCl | N-(3-dimethylaminopropyl)-N'-ethylcarbodiimide hydroch lo ride |
| Et | ethyl |
| Et₃N | triethylamine |
| EtOH | ethanol |
| EtOAc | ethyl acetate |
| g | gram |
| HCl | hydrochloric acid |
| HATU | (1-[Bis(dimethylamino)methylene]-1H-1,2 ,3-triazolo[4,5-b]pyridinium 3-oxid hexafluorophosphate) |
| H₂O | water |
| HPLC | high pressure liquid chromatography |
| Hr | hour |
| IPA | Isopropyl alcohol |
| KHSO₄ | potassium bisulfate |
| K₂CO₃ | Potassium carbonate |
| L | litre |
| LCMS | liquid chromatography mass spectrometry |
| LHMDS | lithium bis(trimethylsilyl)amide |
| LiAlH₄ | lithium aluminium hydride |
| m | multiplet |
| M | molar |
| mBar | millibar |
| MCPBA | 3-chloroperbenzoic acid |
| Me | methyl |
| MeOH | methanol |
| MeOD-d₄ | deutero-methanol |
| 2-MeTHF | 2-methyltetrahydrofuran |
| mg | milligram |
| MHz | mega Hertz |
| mins | minutes |
| mL | millilitres |
| mmol | millimole |
| MS m/z | mass spectrum peak |
| MsCl | methanesulfonyl chloride |
| MTBE | Methyl *tert*-butyl ether |
| M/V | Mass volume ratio |
| N₂ | nitrogen |
| NaBH₄ | sodium borohydride |
| NaHCO₃ | sodium bicarbonate |
| NaOH | sodium hydroxide |
| NBS | N-bromosuccinimide |
| NH₃ | ammonia |
| NH₄Cl | ammonium chloride |
| Na₂SO₄ | sodium sulfate |
| Pd(OH)₂/C | palladium hydroxide on carbon |
| PhSeBr | phenylselenyl bromide |
| PtO₂ | platinum (IV) oxide |
| q | quartet |
| rt | room temperature |
| RT | retention time |
| s | singlet |
| sat. | saturated |
| SCX | strong cation exchange |
| SOCl₂ | Thionyl chloride |
| soln. | solution |
| t | triplet |
| TBAF | *tert*-butyl ammonium fluoride |
| TBME | Methyl tert-butyl ether |
| TFA | trifluoroacetic acid |
| THF | tetrahydrofuran |
| TIPS | triisopropylsilyl chloride |
| TMS | trimethylsilyl |
| T3P | Propylphosphonic anhydride |
| µL | micro litres |
| v/v | volume volume percent |
| w/w | Weight/weight percent |

All of the derivatives of Formula (**I**) can be prepared by the procedures described in the general methods presented below or by routine modifications thereof. The present invention also encompasses any one or more of these processes for preparing the derivatives of Formula (**I**), in addition to any novel intermediates used therein.

The routes below, including those mentioned in the Examples and Preparations, illustrate methods of synthesising the compound of Formula (**I**). The skilled person will appreciate that the compound of the invention, and intermediates thereto, could be made by methods other than those specifically described herein, for example by adaptation of the methods described herein, for example by methods known in the art. Suitable guides to synthesis, functional group interconversions, use of protecting groups, etc., are for example: *"*Comprehensive Organic Transformations" by RC Larock, VCH Publishers Inc. (1989); *"*Advanced Organic Chemistry" by J. March, Wiley Interscience (1985); *"*Designing Organic Synthesis" by S Warren, Wiley Interscience (1978); *"*Organic Synthesis - The Disconnection Approach" by S Warren, Wiley Interscience (1982); *"*Guidebook to Organic Synthesis" by RK Mackie and DM Smith, Longman (1982); *"*Protective Groups in Organic Synthesis" by TW Greene and PGM Wuts, Fifth Ed, John Wiley and Sons, Inc. (2014); and *"*Protecting Groups" by PJ, Kocienski, Georg Thieme Verlag (1994); and any updated versions of these standard works.

In addition, the skilled person will appreciate that it may be necessary or desirable at any stage in the synthesis of compounds of the invention to protect one or more sensitive groups, so as to prevent undesirable side reactions. In particular, it may be necessary or desirable to protect amino or carboxylic acid groups. The protecting groups used in the preparation of the compounds of the invention may be used in a conventional manner. See, for example, those described in 'Greene's Protective Groups in Organic Synthesis' by Theodora W Greene and Peter G M Wuts, fifth edition, (John Wiley and Sons, 2014), in particular Chapter 7 ("Protection for the Amino Group")*,* which also describes methods for the removal of such groups.

In the general synthetic methods below, unless otherwise specified, the substituents are as defined above with reference to the compound of Formula (I), or (I)(a), above.

Where ratios of solvents are given, the ratios are by volume.

The compounds of the invention may be prepared by any method known in the art for the preparation of compounds of analogous structure. In particular, the compound of the invention can be prepared by the procedures described by reference to the Schemes that follow, or by the specific methods described in the Examples, or by similar processes to either.

The skilled person will appreciate that the experimental conditions set forth in the schemes that follow are illustrative of suitable conditions for effecting the transformations shown, and that it may be necessary or desirable to vary the precise conditions employed for the preparation of the compound of Formula (I). It will be further appreciated that it may be necessary or desirable to carry out the transformations in a different order from that described in the schemes, or to modify one or more of the transformations, to provide the desired compound of the invention.

A compound of Formula (I)(a) may be prepared from compounds of Formulae (II)(a), (III)(a), (IV)(a), (V)(a), (X)(a) and (XI)(a) as illustrated by **Scheme 1.** 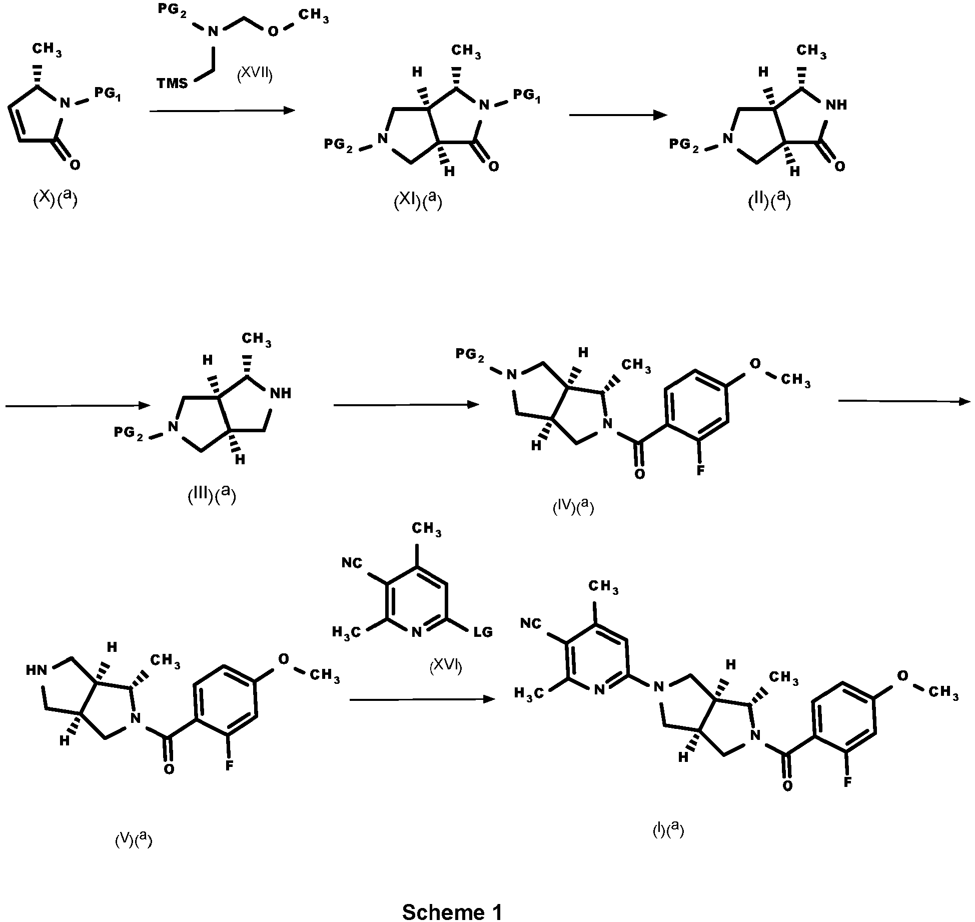 where:
PG₁ and PG₂ are each independently an amine protecting group; LG is a leaving group.

PG₁ and PG₂ may each independently be selected from: a Cbz, MeOZ, ^{t}BOC, FMOC, Ac, Bz, Bn, carbamate, PMB, Ts, Troc, sulphonamide, acetamide, benzamide, *tert*-butylcarbamate, 9-Fluorenylmethyl carbamate, benzhydryl, trityl group.

In particular embodiments, PG₁ is a carbamate group, preferably *t*-butyl carbamate, and PG₂ is benzyl.

Examples of suitable leaving groups include: a chloro, bromo, mesylate, or tosylate group.

In particular embodiments, LG is chloro.

A compound of Formula (XI)(a) (where PG₁ = ^{t}BoC and PG₂ = Bn) may be prepared from a compound of Formula (X)(a), using an acid-mediated cyclisation reaction with N-(methoxymethyl)-N-(trimethylsilylmethyl)benzylamine (XVII) in the presence of a suitable acid, such as TFA, in a suitable aprotic solvent, such as DCM, at a suitable temperature such as 0°C to rt.

A compound of Formula (II)(a) may be prepared by acid mediated deprotection of a compound of Formula (XI)(a) (where PG₁ = ^{t}BoC) using an appropriate acid, such as TFA, in a suitable aprotic solvent, such as DCM, at a suitable temperature such as 0°C to rt.

A compound of Formula (III)(a) may be prepared from a compound of Formula (II)(a) using a suitable reducing agent, such as LiAlH₄, in a suitable aprotic solvent, such as 2-MeTHF, at a suitable temperature, such as between 0°C and reflux, preferably at room temperature.

A compound of Formula (IV)(a) may be prepared from a compound of Formula (III)(a) and 2-fluoro-4-methoxybenzoic acid using an amide bond forming reaction in the presence of a suitable coupling reagent, such as (1-[Bis(dimethylamino)methylene]-1H-1,2,3-triazolo[4,5-b]pyridinium 3-oxid hexafluorophosphate) (HATU) or propylphosphonic anhydride (T3P), in the presence of a suitable organic base, such as DIPEA or Et₃N, in an appropriate aprotic solvent, such as DMF or MeCN, at a suitable temperature, such as 0-100°C, preferably at rt.

Alternatively, a compound of Formula (IV)(a) may be prepared from a compound of Formula (III)(a) and 2-fluoro-4-methoxybenzoyl chloride, prepared in situ from 2-fluoro-4-methoxybenzoic acid, by treatment with a suitable reagent such as SOCl₂ or PCl₃ in a non polar solvent such as toluene at elevated temperature, followed by reaction with a compound of Formula (III)(a), in the presence of a suitable organic base, such as Et₃N or DIPEA in a suitable solvent such as DCM. A skilled person will appreciate that alternative coupling conditions are available in combination with a range of alternative bases and solvents.

A compound of Formula (V)(a) may be prepared from a compound of Formula (IV)(a) (where PG₂ = Bn) using suitable deprotection conditions, such as transfer or palladium catalysed hydrogenation, using for example palladium on charcoal in a suitable solvent, such as EtOH, at a suitable temperature, such as rt, under an atmosphere of hydrogen. A skilled person will appreciate that alternative hydrogenation conditions are available to effect this transformation.

A compound of Formula (I)(a) may be prepared from a compound of Formula (V)(a) and a compound of Formula (XVI) in the presence of a suitable base, such as Cs₂CO₃ or K₂CO₃, in a suitable solvent, such as DMF, DMSO or EtOH, at an elevated temperature, such as 80-100°C.

A compound of Formula (X)(a) may be prepared from compounds of Formulae (VI)(a), (VII)(a), (VIII)(a), and (IX)(a), as illustrated by **Scheme 2:** 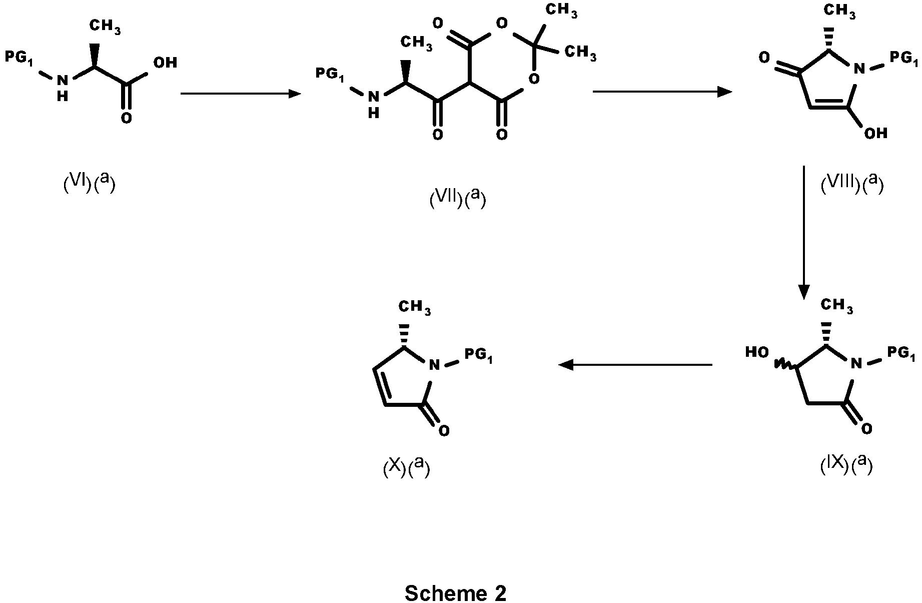 where:
PG₁ is an amine protecting group, as hereinbefore defined.

A compound of Formula (VII)(a) may be prepared by condensation of Boc-L-alanine (VI)(a) and Meldrum's acid in the presence of a suitable condensation reagent, such as EDC.HCI, optionally in the presence of a suitable catalyst, such as DMAP, in a suitable solvent, such as DCM, at a suitable temperature, such as between 0°C and rt.

A compound of Formula (VIII)(a) may be prepared from a compound of Formula (VII)(a) by treatment with heat in a suitable solvent, such as EtOAc, at an appropriate elevated temperature, between 60°C and reflux, optionally at reduced pressure.

A compound of Formula (VIII)(a) may be obtained directly from Boc-L-alanine (VI)(a), without isolating the compound of Formula (VII)(a).

A compound of Formula (IX)(a) may be prepared by the reduction of a compound of Formula (VIII)(a) in the presence of a suitable catalyst such as PtO₂ in the presence of hydrogen gas at a suitable pressure in a solvent such as EtOAc. Alternatively, this transformation may be achieved by treatment of a compound of Formula (VIII)(a) with a suitable reducing agent such as NaBH₄ in the presence of an acid such as AcOH is a suitable solvent such as DCM at between 0°C and room temperature.

A compound of Formula (X)(a) may be prepared from a compound of Formula (IX)(a) using a suitable base-mediated elimination reaction using, for instance an *in situ* conversion of the alcohol into a methansulfonyl leaving group in the presence of a suitable non-nucleophilic base, such as Et₃N, in a suitable solvent, such as DCM, at a suitable temperature, such as 0°C to rt. A skilled person will appreciate that alternative leaving group and elimination conditions are available in combination with a range of alternative bases and solvents.

A compound of Formula (X)(a) may alternatively be prepared from compounds of Formulae (XII)(a), (XIII)(a), (XIV)(a), and (XV)(a) as illustrated by **Scheme 3:** 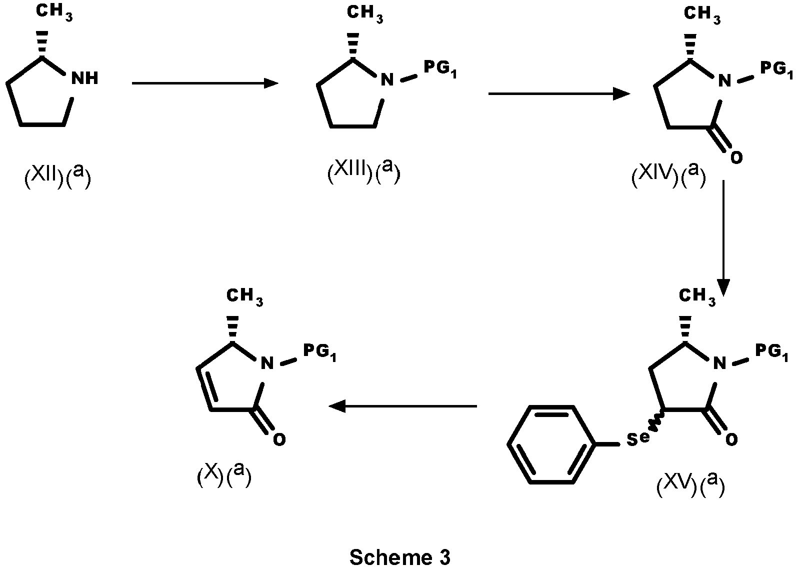 where:
PG₁ is an amine protecting group, as hereinbefore defined.

A compound of Formula (XIII)(a) may be prepared from (2S)-2-methylpyrrolidine hydrochloride (XII)(a) and BOC₂O in a suitable solvent, such as DCM, in the presence of a suitable base, such as Et₃N.

A compound of Formula (XIV)(a) may be prepared by the oxidation of a compound of Formula (XIII)(a) using appropriate oxidising agents such as ruthenium(IV)oxide hydrate and sodium metaperiodate in water at a suitable temperature, such as 15-20°C.

A compound of Formula (XV)(a) may be prepared from a compound of Formula (XIV)(a) by deprotonation using a suitable non-nucleophilic base, such as LHMDS, in a suitable aprotic solvent, such as THF, at low temperature, such as -78°C, followed by quenching with a suitable electrophilic source of phenylselenide, such as PhSeBr, at a suitable temperature, such as rt.

A compound of Formula (X)(a) may be prepared from a compound of Formula (XV)(a) by an oxidative elimination reaction using a suitable oxidising agent, such as MCPBA, in a suitable solvent, such as DCM, at a suitable low temperature, such as -40°C.

A compound of Formula (I)(a) may alternatively be prepared from a compound of Formula (III)(a), (XVIII)(a), (XIX)(a), (XVI), (XX)(a), and (XXI)(a) as illustrated by **Scheme 4:** 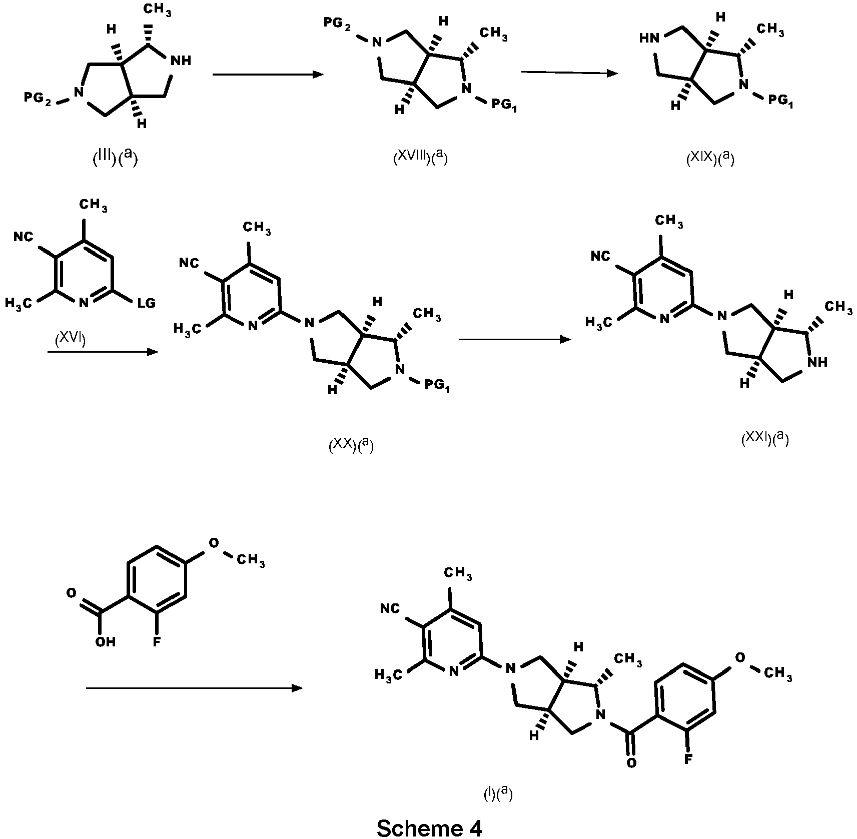 where:
PG₁ and PG₂ are each independently an amine protecting group; LG is a leaving group.

PG₁ and PG₂ may each independently be selected from: a Cbz, MeOZ, ^{t}BOC, FMOC, Ac, Bz, Bn, carbamate, PMB, Ts, Troc, sulphonamide, acetamide, benzamide, *tert-*butylcarbamate, 9-Fluorenylmethyl carbamate, benzhydryl, trityl group.

In particular embodiments, PG₁ is a carbamate group, preferably *t*-butyl carbamate, and PG₂ is benzyl.

Examples of suitable leaving groups include: a chloro, bromo, mesylate, or tosylate group.

In particular embodiments, LG is chloro.

A compound of Formula (XVIII)(a) may be prepared from a compound of Formula (III)(a) under standard conditions for introducing an amine protecting group. Where PG₁ = ^{t}BoC, a compound of Formula (III)(a) is treated with BOC₂O in a suitable solvent such as DCM, typically at room temperature to provide a compound of Formula (XVIII)(a).

A compound of Formula (XIX)(a) may be prepared from a compound of Formula (XVIII)(a) (where PG₂ = Bn) using suitable deprotection conditions, such as transfer or palladium catalysed hydrogenation, using for example ammonium formate and palladium hydroxide on charcoal in EtOH at reflux. A skilled person will appreciate that alternative hydrogenation conditions are available to effect this transformation.

A compound of Formula (XX)(a) may be prepared from a compound of Formula (XIX)(a) and a compound of Formula (XVI) in the presence of a suitable base, such as CS₂CO₃ or K₂CO₃, in a suitable solvent, such as DMF or EtOH, at an elevated temperature, such as 80-110°C.

A compound of Formula (XXI)(a) may be prepared by acid mediated deprotection of a compound of Formula (XX)(a) (where PG₁ = ^{t}BoC) using an appropriate acid, such as TFA, in a suitable aprotic solvent, such as DCM, at a suitable temperature such as 0°C to rt.

A compound of Formula (I)(a) may be prepared from a compound of Formula (XXI)(a) and 2-fluoro-4-methoxybenzoic acid using an amide bond forming reaction in the presence of a suitable coupling reagent, such as (1-[Bis(dimethylamino)methylene]-1H-1,2,3-triazolo[4,5-b]pyridinium 3-oxid hexafluorophosphate) (HATU) or propylphosphonic anhydride (T3P), in the presence of a suitable organic base, such as DIPEA or Et₃N, in an appropriate aprotic solvent, such as DMF or MeCN, at a suitable temperature, such as 0-100°C, preferably at rt. A skilled person will appreciate that alternative coupling conditions are available in combination with a range of alternative bases and solvents.

The skilled person will appreciate that the experimental conditions set forth in the schemes above are illustrative of suitable conditions for effecting the transformations shown, and that they may also be carried out on compounds of the opposite enantiomer to that described or indeed to a racemic mixture. In the latter case a skilled person will also appreciate that individual isomers may be isolated by means of suitable chromatographic conditions.

A compound of Formula (I), (I)(a), (III), (III)(a), (IV), (IV)(a), (V) or (V)(a) may be converted to its corresponding salt using methods known to those skilled in the art. For example, amines of Formula (I), (I)(a), (III), (III)(a), (IV), (IV)(a), (V) or (V)(a) may be treated with trifluoroacetic acid (TFA), HCI, sulfuric acid (H₂SO₄) maleic acid, or citric acid in a solvent such as diethyl ether (Et₂O), CH₂Cl₂, tetrahydrofuran (THF), or methanol (MeOH) to provide the corresponding salt forms.

Compounds prepared according to the schemes described above may be obtained as single enantiomers, diastereomers, or regioisomers, by enantio- , diastero-, or regiospecific synthesis, or by resolution. Compounds prepared according to the schemes above may alternately be obtained as racemic (1:1) or non-racemic (not 1:1) mixtures or as mixtures of diastereomers or regioisomers. Where racemic and non-racemic mixtures of enantiomers are obtained, single enantiomers may be isolated using conventional separation methods known to one skilled in the art, such as chiral chromatography, recrystallization, diastereomeric salt formation, derivatization into diastereomeric adducts, biotransformation, or enzymatic transformation. Where regioisomeric or diastereomeric mixtures are obtained, single isomers may be separated using conventional methods such as chromatography or crystallization.

The following examples are provided to further illustrate the invention and various preferred embodiments.

### Examples

### General methods

¹H nuclear magnetic resonance (NMR) spectra were in all cases consistent with the proposed structures. Characteristic chemical shifts (δ) are given in parts-per-million downfield from tetramethylsilane (for ¹H-NMR) using conventional abbreviations for designation of major peaks: e.g. s, singlet; d, doublet; t, triplet; q, quartet; m, multiplet; br, broad. The following abbreviations have been used for common solvents: CDCl₃, deuterochloroform; DMSO-d₆, hexadeuterodimethyl sulfoxide; and MeOD-d₄, deuteron-methanol. Where appropriate, tautomers may be recorded within the NMR data; and some exchangeable protons may not be visible.

Mass spectra, MS (m/z), were recorded using either electrospray ionisation (ESI) or atmospheric pressure chemical ionisation (APCI). Where relevant and unless otherwise stated the m/z data provided are for isotopes ¹⁹F, ³⁵Cl, ⁷⁹Br and ¹²⁷I.

Where preparative TLC or silica gel chromatography have been used, one skilled in the art may choose any combination of solvents to purify the desired compound.

### Preparation 1

### tert-Butyl (2S)-5-hydroxy-2-methyl-3-oxo-2,3-dihydro-1H-pyrrole-1-carboxylate

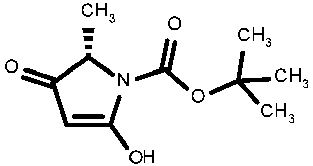

In to a 20 L reactor was charged DCM (5.2 L) followed by Boc-L-alanine (650 g, 3.43 mol), Meldrum's acid (545 g, 3.77 mol) and DMAP (630 g, 5.15 mol) and the line washed with further DCM (1.3 L). The solution was cooled to 0 °C, then EDC·HCl (790 g, 4.122 mol) was added portion wise over 20 mins and the reaction stirred at rt for 3.5 hr. The solution was washed with 1M citric acid soln. (4x 3.9 L) and water (3.9 L). The organic solution was concentrated to ca. 2.6 L, diluted with EtOAc (6.5 L) then further concentrated to ca. 2.6 L. EtOAc (3.9 L) was added again and the solution maintained at 0°C for 18 hrs. The solution was heated to 80°C with an internal vacuum of 800 mBar in distillation mode (internal temperature 66-67°C) and stirred for 1.5 hr. The solvent was evaporated to minimum volume (1 L) and the solution used in the next step without further purification. A drop of this solution was evaporated to obtain an analytical sample.
LCMS m/z =212.2 [M-H]⁻

### Preparation 2

### tert-Butyl (2S)-3-hydroxy-2-methyl-5-oxopyrrolidine-1-carboxylate

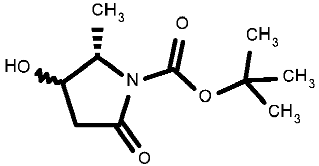

The solution of *tert*-butyl (2S)-5-hydroxy-2-methyl-3-oxo-2,3-dihydro-1H-pyrrole-1-carboxylate (Preparation 1, 732 g, 3.43 mol) was diluted with DCM (5.1 L) and AcOH (2.2 L), and the mixture cooled to 0 °C. NaBH₄ (195 g, 5.15 mol) was added portion wise over 6 hrs, water (7.3 L) was carefully added to quench the reaction and the mixture stirred for 15 hrs. The phases were separated, the DCM layer was collected and the aqueous phase washed with DCM (0.7 L x4). The combined organic layers were filtered through a Gooch filter, then AcOH (2.2 L) added and the solution cooled to 0 °C. NaBH₄ (97.5 g, 2.57 mol) was added portion wise over 1 hr and the resulting thick suspension stirred for 30 mins. K₂CO₃ (solution 40% M/V) (7.3 L) was added and the two phases were separated. The water layer was extracted with DCM (1 Lx 2) and the combined organic phases were dried (Na₂SO₄) and filtered to obtain a solution which was used in the next step without further purification. 10 mL of this solution was evaporated to dryness to obtain 1.625 g of product (estimated yield 650 g, 88%).
¹HNMR (DMSO-d₆, 400MHz) δ : 1.18 (d, 3H), 1.45 (s, 9H), 2.35-2.57 (ddd, 2H), 4.05 (quintet, 1H), 4.24-4.29 (m, 1H), 5.30 (br s, 1H).

### Preparation 3

### tert-Butyl (2S)-2-methyl-5-oxo-2,5-dihydro-1H-pyrrole-1-carboxylate

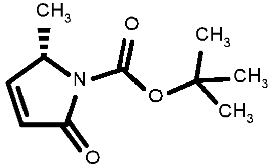

Et₃N (1.06 L, 7.63 mol) was added to a solution of *tert*-butyl (2S)-3-hydroxy-2-methyl-5-oxopyrrolidine-1-carboxylate (Preparation 2, 651 g, 3.05 mol) in DCM (6.5 L) and the mixture cooled to 0 °C. MsCI (283 mL, 3.66 mol) was added dropwise over 2 hrs, and the reaction then allowed to warm to rt, and stirred for 2 hrs. The mixture was washed with water (3 x 2L). The organic solution obtained was used in the following step without any further purification. 10 mL of this solution was evaporated to dryness to obtain 0.843 g of product (estimated yield 590 g, 98%). ¹HNMR (DMSO-d₆, 400MHz) δ : 1.35 (d, 3H), 1.48 (s, 9H), 4.58-4.65 (m, 1H), 6.08 (dd, 1H), 7.43 (dd, 1H).

### Preparation 4

### tert-Butyl (1S,3aR,6aS)-5-benzyl-1-methyl-3-oxo-octahydropyrrolo[3,4-c]pyrrole-2-carboxylate

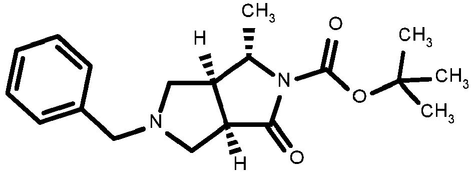

To an ice-cooled stirred solution of *tert*-butyl (2S)-2-methyl-5-oxo-2,5-dihydro-1H-pyrrole-1-carboxylate (Preparation 3, 590 g, 2.99 mol) in DCM (5.9 L) and TFA (62 mL, 0.81 mol) was added N-(methoxymethyl)-N-(trimethylsilylmethyl) benzylamine (1721 mL, 6.73 mol) dropwise over 4 hrs and the mixture then stirred at rt for 15 hrs. The reaction mixture was partitioned between DCM and saturated NaHCO₃ soln. (5.9 L), the layers separated and the aqueous phase extracted twice with DCM (2x 1.2 L). The combined organic layers were evaporated, the residue diluted with EtOAc (3.0 L) and the solution washed with aq. NH₄Cl soln. (3x 3.0 L at pH ∼5) and once with water (3.0 L). The solvent was concentrated to minimum volume (∼ 2L) under vacuum to afford an orange oil (1.9 Kg) which was taken up in Cy:EtOAc 95:5 (3.8 L) and stirred for 60 hrs. The resulting solid formed was filtered off to afford the title compound as an off-white solid, (470 g, 49%).

The mother liquors were evaporated to dryness and the orange oil (1.3 Kg) was passed through a SiO₂ pad (1.2 Kg) eluting with Cy (2.4 L, [discarded]) then Cy:EtOAc 50:50 (6.0 L). The solution collected was evaporated and the residue taken up in EtOAc (590 mL) and charged into a reactor. The solvent was concentrated to a minimum volume, Cy (1.2 L) was added and the mixture stirred at rt for 15 hrs. The slurry was filtered under vacuum, washed with Cy (200 mL) and oven dried to obtain additional title compound as a white solid, (140 g, 14%).

The mother liquors were evaporated to dryness and the orange oil (270 g) taken up in Cy (0.6 L) and stirred for 15 hrs at rt. The solid formed was filtered under vacuum and washed with Cy (4x 25 mL portions) to obtain further title compound as a white solid, (60 g, 6%).
LCMS m/z = 331.3 [MH]⁺

### Alternative preparation

### tert-Butyl (1S,3aR,6aS)-5-benzyl-1-methyl-3-oxo-octahydropyrropo[3,4-c]pyrrole-2-carboxylate

To an ice-cooled stirred solution of *tert*-butyl (2S)-2-methyl-5-oxo-2,5-dihydro-1H-pyrrole-1-carboxylate (Preparation 3, 845 g, 4.28 mol) in DCM (8.45 L) and TFA (89 mL, 1.15 mol) was added N-(methoxymethyl)-N-(trimethylsilylmethyl) benzylamine (1450 mL, 5.66 mol) dropwise over 1 hr and the mixture then stirred at 10°C for 15 hrs. The reaction mixture was partitioned between DCM and saturated NaHCO₃ soln. (8.45 L), the layers separated and the aqueous phase extracted twice with DCM (2x 1.69 L). The combined organic layers were evaporated to a minimum volume, the residue diluted with EtOAc (4.2 L) and the solution washed with aq. NH₄Cl soln. (3x 4.2 L at pH ∼5) and water (4.2 L). The solvent was concentrated to minimum volume (∼ 2L) under vacuum to afford an orange oil (2.0 Kg) which was taken up in Cy:EtOAc 95:5 (5.3 L). An authentic sample of product was added to promote crystallisation and the mixture stirred for 60 hrs. The resulting solid that formed was filtered off to afford the title compound as an off-white solid, (290 g, 20%).

The mother liquors were evaporated to dryness and the orange oil (1.7 Kg) was passed through a SiO₂ pad (1.5 Kg) eluting with Cy (3.4 L, [discarded]) then Cy:EtOAc 50:50 (8.45 L). The solution collected was evaporated, the residue charged into a reactor and Cy (2.0 L) added. This solution was filtered through SiO₂ eluting with Cy:EtOAC 50:50 (5.8 L), the solution collected was concentrated under reduced pressure and charged into the reactor washing with Cy (300 mL). The mixture was concentrated to ca. 2.5 L and the suspension stirred at rt for 15 hrs. The slurry was filtered under vacuum, washed with Cy (500 mL) and oven dried to obtain the title compound as an off-white solid, 507 g, 36%.

The mother liquors were evaporated to dryness and the orange oil taken up in Cy (0.85 L) and stirred for 15 hrs at rt. The solid formed was filtered under vacuum and washed with Cy (4x 35 mL portions) to obtain further title compound as a white solid, (64 g, 6%). Total yield 861 g, 61% yield.
¹HNMR (400MHz, DMSO-d₆) δ: 1.27 (d, 3H), 1.47 (s, 9H), 2.33-2.41 (m, 2H), 2.45-2.49 (m, 1H), 2.71 (dd, 1H), 2.92 (d, 1H), 3.10-3.15 (m, 1H), 3.54 (dd, 2H), 3.84-3.90 (m, 1H), 7.22-7.26 (m, 3H), 7.29-7.34 (m, 2H).
LCMS m/z = 331.4 [MH]⁺

### Preparation 5

### (3S,3aS,6aR)-5-Benzyl-3-methyl-octahydropyrrolo[3,4-c]pyrrol-1-one

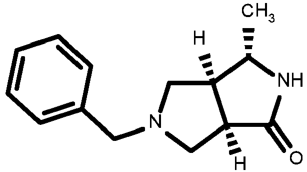

TFA (1.423 L, 18.58 mol) was added to a solution of *tert*-butyl (1S,3aR,6aS)-5-benzyl-1-methyl-3-oxo-octahydropyrrolo[3,4-c]pyrrole-2-carboxylate (Preparation 4, 470 g, 1.422 mol) in DCM (4.7 L) and the reaction stirred at rt for 15 hrs. The reaction was cooled to -5 °C and freshly prepared 6M NaOH soln. was added dropwise keeping the internal temperature below 15 °C to neutralise the solution. Once addition was complete, EtOAc (300 mL) was added and the two phases were separated. The organic phase was washed with water (2 L) then passed through a thin layer of Celite® to break the emulsion. The organic solution was washed again with water (2 L) filtered through a thin layer of Celite® then charged into a reactor. The solution was cooled to 0 °C and allowed to stand for 15 hrs. The temperature was increased to 20 °C, the solvent evaporated to dryness, then azeotroped with 2-MeTHF to obtain the title compound as a dark orange oil, 380 g, in quantitative yield.
LCMS m/z = 231.0 [MH]⁺

### Alternative Preparation

### (3S,3aS,6aR)-5-Benzyl-3-methyl-octahydropyrrolo[3,4-c]pyrrol-1-one

TFA (1.9 L, 24.81 mol) was added to a solution of *tert*-butyl (1S,3aR,6aS)-5-benzyl-1-methyl-3-oxo-octahydropyrrolo[3,4-c]pyrrole-2-carboxylate (Preparation 4, 997 g, 3.017 mol) in DCM (1.0 L) and the reaction stirred at rt for 30 mins. The solvent was evaporated *in vacuo* and the TFA co-distilled with toluene. The residue was taken up in DCM (10 L), washed with sat. NaHCO₃ soln. (10 L), the phases separated and the aqueous extracted with DCM (2 x 2 L). The combined organic solutions were washed with water (10 L), then evaporated *in vacuo* and the residue azeotroped with 2-MeTHF (2 L) to afford the title compound as a dark orange oil in quantitative yield.
¹HNMR (400MHz, DMSO-d₆) δ: 1.12 (d, 3H), 2.23-2.36 (m, 2H), 2.49-2.52 (m, 1H), 2.67 (dd, 1H), 2.77-2.83 (m, 1H), 2.85-2.88 (m, 1H), 3.24-3.30 (m, 1H), 3.52-3.54 (m, 2H), 7.21-7.35 (m, 5H), 7.63 (br s, 1H).

### Preparation 6

### (1S,3aS,6aS)-5-Benzyl-1-methyl-octahydropyrrolo[3,4-c]pyrrole

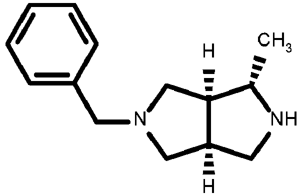

LiAlH₄ pellets (133 g, 3.56 mol) were carefully added to 2-MeTHF (2.6 L) and the dark suspension was stirred at rt for 15 hrs. A solution of (3S,3aS,6aR)-5-benzyl-3-methyl-octahydropyrrolo[3,4-c]pyrrol-1-one (Preparation 5, 327 g, 1.422 mol) in 2-MeTHF (660 mL) was added dropwise at 0 °C over 1 hr and the resulting reaction was heated under reflux for 3 hrs. The reaction was cooled to -10°C, then water (135 mL), NaOH (15% w/w, 135 mL) and water (405 mL) were slowly added in this order over 2 hrs. The white precipitate obtained was stirred for 15 hrs at rt, filtered and washed with 2-MeTHF (2 L). The filtrate was evaporated to dryness to afford the title compound as a clear orange oil, 280 g, 91%.
LCMS m/z = 217.3 [MH]⁺

### Preparation 6A

### (1S,3aS,6aS)-5-Benzyl-1-methyl-octahydropyrrolo[3,4-c]pyrrole hydrochloride

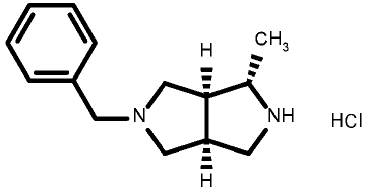

6M HCI in IPA (56.67 mL, 340 mmol) was added dropwise to a solution of (1S,3aS,6aS)-5-benzyl-1-methyl-octahydropyrrolo[3,4-c]pyrrole (Preparation 6, 78 g, 351 mmol) in TBME (200 mL) at 0 °C. The mixture was stirred at rt for 30 mins. The resulting solid was filtered under vacuum to obtain the title compound as a beige solid (50 g, 56 %).

### Preparation 7

### (1S,3aR,6aS)-5-Benzyl-2-(2-fluoro-4-methoxybenzoyl)-1-methyl-octahydropyrrolo[3,4-c]pyrrole

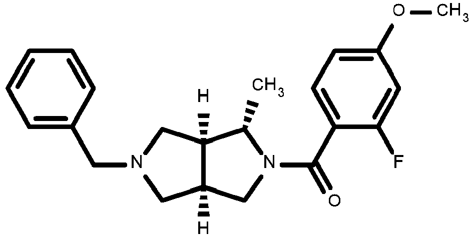

2-Fluoro-4-methoxybenzoic acid (35g, 204 mmol) was suspended in SOCl₂ (70 mL) and the mixture stirred at 80 °C for 5 hr. The mixture was evaporated under vacuum and azeotroped with toluene (3x50 mL). The resulting oil was dissolved in DCM (300 mL), cooled to 0 °C and Et₃N (85 mL, 582 mmol) added. (1S,3aS,6aS)-5-Benzyl-1-methyl-octahydropyrrolo[3,4-c]pyrrole hydrochloride (Preparation 6a, 49.2 g, 194 mmol) was added portion wise over 5 mins and the reaction then stirred for 30 mins. Sat. NaHCO₃ soln. (300 mL) was added and the mixture was stirred for 30 mins. The organic phase was separated, additional sat. NaHCO₃ soln. (200 mL) added and the mixture stirred for 18 hrs. The organic phase was separated, dried (Na₂SO₄) and evaporated under reduced pressure to afford the title compound.
LCMS m/z = 369.0 [MH]⁺

### Alternative preparation

### (1S,3aR,6aS)-5-Benzyl-2-(2-fluoro-4-methoxybenzol)-1-methyl-octahydropyrrolo[3,4-c]pyrrole

_(1S,3aS,6aS)-5-Benzyl-1-methyl-octahydropyrrolo[3,4-c]pyrrole (Preparation 6, 578 g, 2.672 mol) was added to a solution of 2-fluoro-4-methoxybenzoic acid (500 g, 2.939 mol) and Et₃N (1.19 L, 8.55 mol) in MeCN (5. 8L) and the mixture cooled to 0°C. T3P (2040 g, 3.206 mol) was added dropwise over 45 mins and the reaction stirred at rt for 1 hr. The mixture was concentrated to ca. 1.7 L, EtOAc (5.8 L) added and the solution washed twice with 20% (w/w) aq. K₂CO₃ (2x 5.8 L) and once with a solution 1:1 H₂O/brine solution (5.8 L). The solvent was evaporated *in vacuo* and the residue suspended in EtOH (2.9 L). The solution was evaporated to dryness to obtain the title compound as a dark oil that was used in the next step without further purification.
¹HNMR (400MHz, DMSO-d₆) (mixture of rotamers) δ: 0.97, 1.17 (d, 3H), 2.11-2.29 (m, 2H), 2.35-2.55 (m, 2H), 2.65-2.71 (m, 1H), 2.72-3.06 (m, 2H), 3.42-3.59 (m, 3H), 3.80 (s, 3H), 4.26-4.28 (m, 1H), 6.81-6.94 (m, 2H), 7.21-7.35 (m, 6H).
LCMS m/z = 369.1 [MH]⁺

### Preparation 8

### (1S,3aR,6aS)-2-(2-Fluoro-4-methoabenzoyl)-1-methyl-octahydropyrrolo[3,4-c]pyrrole

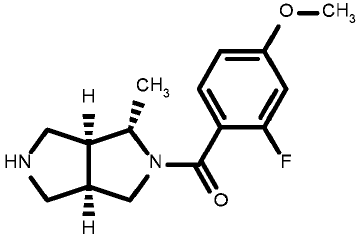

(1S,3aR,6aS)-5-Benzyl-2-(2-fluoro-4-methoxybenzoyl)-1-methyl-octahydropyrrolo[3,4-c]pyrrole (Preparation 7, 75.5 g, 202 mmol) was dissolved in EtOH (1 L), 10% Pd/C (11 g, 10% w/w) was added and the solution stirred under H₂ atmosphere (6 bar) for 20 hrs. The mixture was filtered through a pad of Celite® and the solvent was evaporated *in vacuo* to obtain the title compound as a sticky oil, 56 g, 99%.
LCMS m/z = 279.0 [MH]⁺

### Example 1

### 6-[(3aS,4S,6aR)-5-(2-fluoro-4-methoxybenzoyl)-4-methyl-octahydropyrrolo[3,4-c]pyrrol-2-yl]-2,4-dimethylpyridine-3-carbonitrile

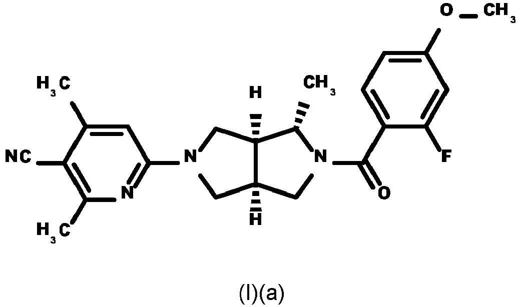

6-Chloro-2,4-dimethylnicotinonitrile (198 mg, 1.18 mmol) and K₂CO₃ (164 mg, 1.19 mmol) were added to a solution of (1S,3aR,6aS)-2-(2-fluoro-4-methoxybenzoyl)-1-methyl-octahydropyrrolo[3,4-c]pyrrole (Preparation 8, 300 mg, 1.08 mmol) in EtOH (3 mL) and the reaction stirred at 80°C for 3 hrs. The cooled mixture was partitioned between DCM and water, the organic phase was separated and evaporated *in vacuo.* The crude material was suspended in MTBE (10 mL) and EtOAc (1 mL) and the mixture stirred at rt for 18 hrs. The resulting solid was filtered off and washed with cyclohexane (3 mL) to afford 268 mg, 61% of the title compound. The mother liquor were concentrated *in vacuo,* and purified by column chromatography on silica gel eluting with cy:EtOAc (100:0 to 0:100) to obtain an additional 84 mg, 19% of the title compound.
¹HNMR (400MHz, MeOD-d₄) (mixture of rotamers) δ: 1.12, 1.44 (2xd, 3H), 2.38, 2.40 (2xs, 3H), 2.52, 2.54 (2xs, 3H), 2.79-2.84 (m, 1H), 3.12- 3.35 (m, 2H), 3.49-4.24 (m, 9H), 6.28, 6.31 (2xs, 1H), 6.77-6.89 (m, 2H), 7.29-7.35 (m, 1H)
LCMS m/z = 409.1 [MH]⁺

### Alternative preparation

### 6-[(3aS,4S,6aR)-5-(2-Fluoro-4-methoxybenzoyl)-4-methyl-octahydropyrrolo[3,4-c]pyrrol-2-yl]-2,4-dimethylpyridine-3-carbonitrile

A 20 L reactor was charged with (1S,3aR,6aS)-2-(2-fluoro-4-methoxybenzoyl)-1-methyl-octahydropyrrolo[3,4-c]pyrrole (Preparation 8, 625 g, 2.24 mol) and DMSO (1.87 L) at 25°C. 6-Chloro-2,4-dimethylnicotinonitrile (344 g, 2.06 mol) and K₂CO₃ (466 g, 3.37 mol) were added, the lines washed with DMSO (1.23 L) and the reaction stirred at 70°C for 17 hrs. The cooled suspension was filtered on a Gooch filter, washing through with additional DMSO (625 mL). The reactorwas charged with water (12.5 L) and the combined organic solutions added drop wise over 2 hrs with vigorous stirring. The suspension was heated to 60°C, stirred for 30 mins then cooled to 25°C and after 30 mins heated again to 60°C. After 30 min the suspension was cooled again to 25°C and stirred for 8 hrs. The mixture was filtered (3x) and the solid washed with water (3 x 6.25 L). The filter cakes were dried under vacuum at 50°C for 4 days to afford the title compound as a solid, 770 g, 90%.
¹HNMR (400MHz, MeOD-d₄) (mixture of rotamers) δ: 1.12, 1.44 (2xd, 3H), 2.38, 2.40 (2xs, 3H), 2.52, 2.54 (2xs, 3H), 2.79-2.84 (m, 1H), 3.12- 3.35 (m, 2H), 3.49-4.24 (m, 9H), 6.28, 6.31 (2xs, 1H), 6.77-6.89 (m, 2H), 7.29-7.35 (m, 1H)
LCMS m/z = 409.1 [MH]⁺

### Example 2

### In vitro affinity of the compound of Example 1 for human orexin-1 and orexin-2 receptors

The *in vitro* binding affinity of 6-[(3aS,4S,6aR)-5-(2-fluoro-4-methoxybenzoyl)-4-methyl-octahydropyrrolo[3,4-c]pyrrol-2-yl]-2,4-dimethylpyridine-3-carbonitrile 3278/6 (the compound of Example 1) for_human recombinant OX1 (hOX1R) and OX2 (hOX2R) receptors was determined using a Scintillation Proximity binding Assay (SPA).

A 96-well plate SPA was developed using antagonist radioligands (OX-radioligands); [3H]-SB674042 (Langmead *et al.,* 2004; Malherbe *et al.,* 2009a) for **hOX1R** and [3H]-EMPA (Malherbe *et al.,* 2009b) for **hOX2R.** Cell membranes were prepared from recombinant cell lines; a CHO cell line transformed with **hOX1R** (GeneBank accession# NM_001525) and a HEK cell line transformed with **hOX2R** (GeneBank accession# NM_001526.2).

On the day of the assay, membranes containing hOX1R or hOX2R were captured onto SPA-beads. All test compounds were dissolved in 100% DMSO as 10mM stock solutions, and prepared as 8-point semi-log dilution in 100% DMSO immediately prior to assay (final assay range 5uM to 0.064nM). The compound of Example 1 was co-administered with the appropriate OX-radioligand, and the SPA-membrane preparation added to initiate the assay. During the assay, any compound of Example 1 that bound to the receptors competed with radioligand and reduced the assay signal. Assay plates were read using PerkinElmer Microbeta2 allowing the binding profile of the compound of Example 1 to be assessed at room temperature (19°C) over several time points (15, 60, 90, 120, 150 minutes, and 18 hours) in hOX1R and hOX2R assay formats.

### Data Handling and Analysis.

*End-point data values* were determined as Specific Binding (SB) and expressed as a percentage of control (%SB), with 100% being the SB of OX-radioligand in the absence of competing unlabelled ligand, and 0% being the SB of the relevant OX-radioligand in the presence of an excess of the competing cold/unlabelled ligand (Suvorexant; a dual OXR-antagonist [DORA]).
TB= Total Bound (in presence of 0.5% DMSO);
NSB= Non Specific Bound (in the presence of Suvorexant at 1 µM) = 0%;
SB= Specific Bound (TB-NSB) = 100%

### Curve fitting and Affinity determination

Assay data were used to determine half maximal inhibitory concentration (IC₅₀) using a four-parameter logistic model in XLfit and/or GraphPad Prism v5. IC₅₀ were derived from plots of %SB against log10 of the concentration of the compound of Example 1. The pKi were calculated from the IC₅₀ using the Cheng-Prusoff correction: pKi = IC₅₀ /(1+([L]/KD)) where [L] is the radioligand concentration in the displacement assay, and KD is the dissociation constant of the radioligand. The Selectivity value is derived from the Ki [Ki (OX2R)/Ki(OX1R)].

### Results

The results are shown in tables 1 and 2 below:

**Table 1**

| **Compound** | **Batches** | **IC₅₀ OX1R (M)** | **IC₅₀ OX2R (M)** | **OX1R pIC₅₀*** | **OX2R pIC₅₀*** | **Selectivity OX2R/OX1R**** | **n** |
|---|---|---|---|---|---|---|---|
| Example 1 **GeoMean** | A/2668/6/2 | 9.77E-8 | 1.23E-9 | 7.01 | 8.91 | 69 | 5 |

| | | | | | | | |
|---|---|---|---|---|---|---|---|
| * data at 60min ** selectivity calculated using Ki (Ox2R)/Ki(Ox1 R) | | | | | | | |

**Table 2**

| **Compound** | **hOX1R Binding pKi (60 min)** | **hOX2R Binding pKi (60 min)** | **hOX2R Binding Equilibration Time (minutes)** | **hOX2R Selectivity (IC50 hOX2R/hOX1R)** |
|---|---|---|---|---|
| Example 1 | 7.32 | 9.14 | 38 | 69 |

| | | | | |
|---|---|---|---|---|
| Data: geometric mean, derived from five duplicate runs | | | | |

The results show that the compound of Example 1 has high affinity for hOX1R and hOX2R, with 69-fold selectivity for hOX2R.

### Example 3

### In vitro affinity of the compound of comparative Example A for human orexin-1 and orexin-2 receptors

### 6-[(3aS,4S,6aR)-5-(2-fluoro-4-methoxybenzoyl)-octahydropyrrolo[3,4-c]pyrrol-2-yl]-2,4-dimethylpyridine-3-carbonitrile (comparative Example A)

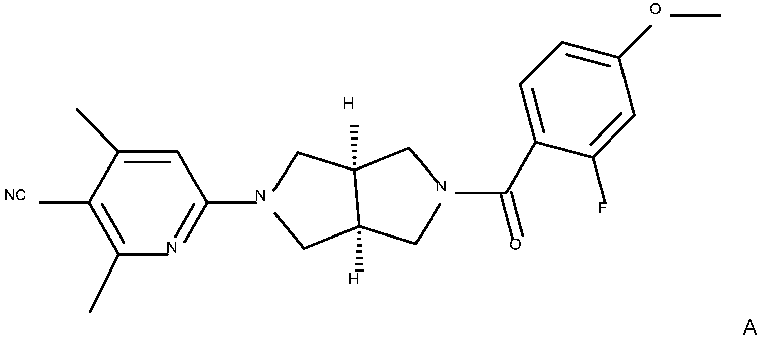

was prepared and tested by analogous methods to those described above in Example 1 and Example 2 in respect of the compound of Example 1.

The results for comparative example A are shown in Tables 3 and 4 below

**Table 3**

| **Compound** | **Batches** | **IC₅₀ OX1R (M)** | **IC₅₀ OX2R (M)** | **OX1R pIC₅₀*** | **OX2R pIC₅₀*** | **OX2R Selectivity (IC50 OX1R/OX2R)** | n |
|---|---|---|---|---|---|---|---|
| Example A | A/2781/30/2 | 7.84E-6 | 1.50E-7 | 5.11 | 6.82 | 52 | 5 |

| | | | | | | | |
|---|---|---|---|---|---|---|---|
| * data at 60min | | | | | | | |

**Table 4**

| **Compound** | **hOX1R Binding pKi (60 min)** | **hOX2R Binding pKi (60 min)** | **hOX2R Binding Equilibration Time (minutes)** | **Selectivity hOX2R/hOX1R**** |
|---|---|---|---|---|
| Example A | 5.44 | 7.07 | nd | 43 |

| | | | | |
|---|---|---|---|---|
| Data: geometric mean, derived from 4 duplicate runs ** selectivity calculated using Ki(Ox2R)/Ki(Ox1 R) | | | | |

The results show that the compound of Example 1 has higher affinity for hOX2R than comparative Example A. The compound of Example 1 differs from comparative Example A of by virtue of 4-methyl substitution of octahydropyrrolo[3,4-c]pyrrol-2-yl moiety. The results therefore evidence that this methyl substitution gives rise to the increased affinity at hOX2R when compared to comparative Example A.

### References

Langmead C.J., Jerman J.C., Brough S.J., Scott C., Porter R.A., Herdon H.J. (2004). Characterisation of the binding of [3H]-SB-674042, a novel nonpeptide antagonist, to the human orexin-1 receptor. Br. J. Pharmacol. , 141: 340-346;
Malherbe P., Borroni E., Pinard E., Wettstein j.G., and Knoflach F. (2009a) Biochemical and Electrophysiological Characterization of Almorexant, a Dual Orexin Receptor (OX1)/Orexin 2 Receptor (OX2) Antagonist: Comparison with Selective OX1 and OX2 Antagonists. Mol. Pharmacol. 76, 618-631;
Malherbe P., Borroni E., Gobbi L., Knust H., Nettekoven M., Pinard E., Roche O., Rogers-Evans M., Wettstein J.G. and Moreauet J-L, (2009b) Biochemical and behavioural characterization of EMPA, a novel high-affinity, selective antagonist for the OX2 receptor. Br. J. Pharmacol. 156, 1326-1341.

## Claims

1. A compound of Formula (I): 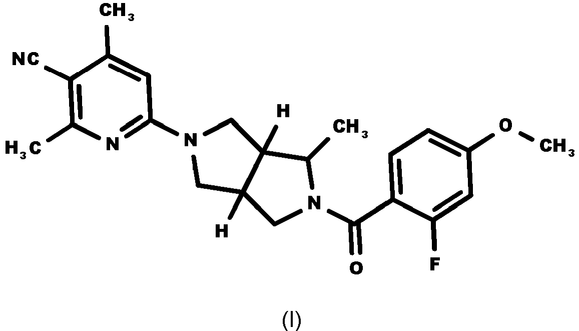 or a pharmaceutically acceptable salt, solvate, hydrate, geometrical isomer, tautomer, or optical isomer thereof.

2. A compound according to claim 1, wherein the central hydrogen atoms on the methylated octahydropyrrolo[3,4-c]pyrrole group are in a *cis* position.

3. A compound according to claim 1 or 2, wherein the compound is of Formula (I)(a): 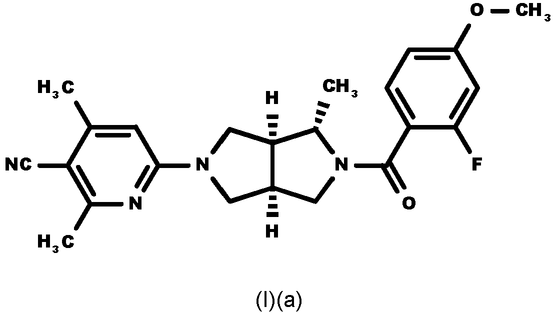 or a pharmaceutically acceptable salt, solvate, hydrate, geometrical isomer, tautomer, or optical isomer thereof.

4. A pharmaceutical composition comprising a compound according to any of claims 1 to 3, and a pharmaceutically acceptable excipient.

5. A compound according to any of claims 1 to 3, or a composition according to claim 4, for use as a medicament.

6. A compound according to any of claims 1 to 3, or a composition according to claim 4, for use in treating a disease, disorder or condition mediated by orexin receptor activity, wherein the disease, disorder, or condition is selected from the group consisting of: a disorder of the sleep-wake cycle, a metabolic disorder, a neurological disorder, and other disorders, such as, feeding, drinking, arousal, stress, addiction, especially drug addiction, metabolism and reproduction disorders.

7. A compound according to any of claims 1 to 3, or a composition according to claim 4, for use in treating a disease, disorder or condition mediated by orexin receptor activity, wherein the disease, disorder, or condition is selected from the group consisting of: disorders of the sleep-wake cycle, sleep-wake transition disorder, insomnia, restless legs syndrome, jet-lag, disturbed sleep, sleep disorders secondary to neurological disorders, manias, depressions, manic depression, schizophrenia, pain syndromes, fibromyalgia, neuropathic pain, catatonia, Parkinson's disease, Tourette's syndrome, anxiety, stress disorder, panic disorder, delirium, dementias, Alzheimer's disease, Huntington's disease, overweight or obesity, conditions related to overweight or obesity, insulin resistance, type II diabetes, hyperlipidemia, gallstones, angina, hypertension, breathlessness, tachycardia, infertility, sleep apnoea, back and joint pain, varicose veins, osteoarthritis, hypertension, tachycardia, arrhythmias, angina pectoris, acute heart failure, ulcers, irritable bowel syndrome, diarrhoea, gastroesophageal reflux, addiction, and alcoholism.

8. A compound according to any of claims 1 to 3, or a composition according to claim 4, for use in treating a disease, disorder or condition mediated by orexin receptor activity, wherein the disease, disorder, or condition is a disorder of the sleep-wake cycle, sleep-wake transition disorder, or insomnia.

9. Use of a compound as defined in any of claims 1 to 3, or a composition as defined in claim 4, in the manufacture of a medicament for treating a disease, disorder or condition mediated by orexin receptor activity, wherein the disease, disorder, or condition is selected from the group consisting of: a disorder of the sleep-wake cycle, a metabolic disorder, a neurological disorder, and other disorders, such as, feeding, drinking, arousal, stress, addiction, especially drug addiction, metabolism and reproduction disorders.

10. Use of a compound as defined in any of claims 1 to 3, or a composition as defined in claim 4, in the manufacture of a medicament for treating a disease, disorder or condition mediated by orexin receptor activity, wherein the disease, disorder, or condition is selected from the group consisting of: disorders of the sleep-wake cycle, sleep-wake transition disorder, insomnia, restless legs syndrome, jet-lag, disturbed sleep, sleep disorders secondary to neurological disorders, manias, depressions, manic depression, schizophrenia, pain syndromes, fibromyalgia, neuropathic pain, catatonia, Parkinson's disease, Tourette's syndrome, anxiety, stress disorder, panic disorder, delirium, dementias, Alzheimer's disease, Huntington's disease, overweight or obesity, conditions related to overweight or obesity, insulin resistance, type II diabetes, hyperlipidemia, gallstones, angina, hypertension, breathlessness, tachycardia, infertility, sleep apnoea, back and joint pain, varicose veins, osteoarthritis, hypertension, tachycardia, arrhythmias, angina pectoris, acute heart failure, ulcers, irritable bowel syndrome, diarrhoea, gastroesophageal reflux, addiction, and alcoholism.

11. Use of a compound as defined in any of claims 1 to 3, or a composition as defined in claim 4, in the manufacture of a medicament for treating a disease, disorder or condition mediated by orexin receptor activity, wherein the disease, disorder, or condition is a disorder of the sleep-wake cycle, sleep-wake transition disorder, or insomnia.

12. A method of producing a compound of Formula (I), which comprises reacting a compound of Formula (V) and a compound of Formula (XVI), to produce the compound of Formula (I): 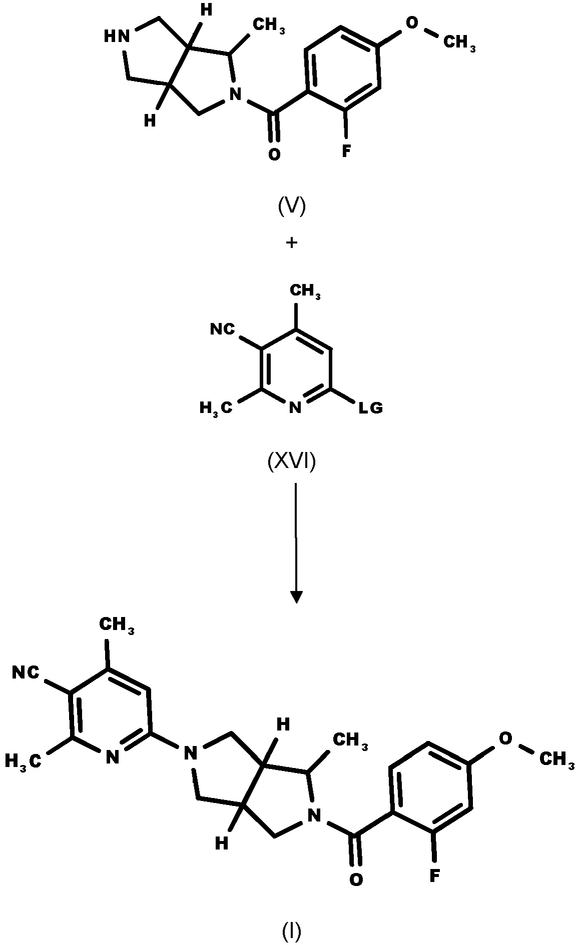 where LG is a leaving group, such as a chloro, bromo, mesylate, or tosylate group, preferably chloro.

13. A method according to claim 12, wherein the central hydrogen atoms on the methylated octahydropyrrolo[3,4-c]pyrrole group in compound (V) and compound (I) are in a *cis* position.

14. A method according to claim 12 or 13, which comprises reacting a compound of Formula (V)(a) and a compound of Formula (XVI), to produce a compound of Formula (I)(a): 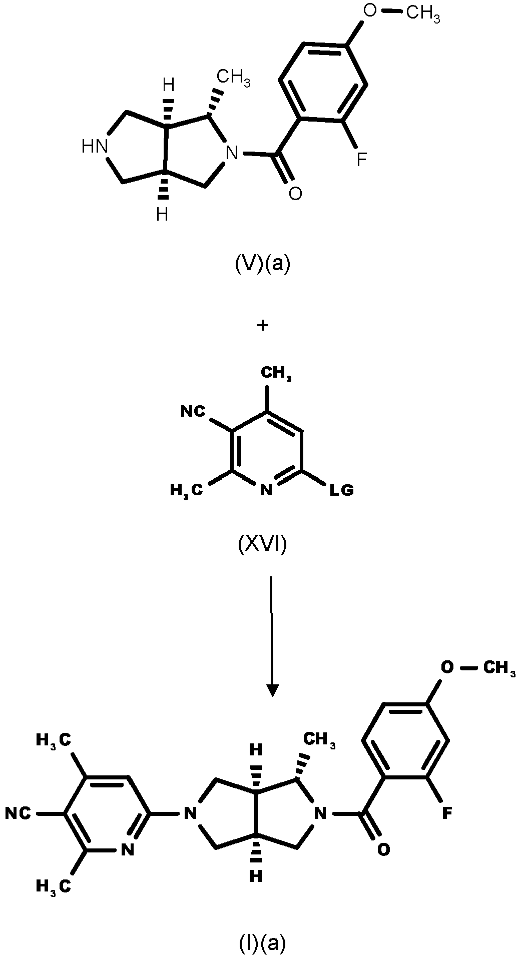 where LG is a leaving group, such as a chloro, bromo, mesylate, or tosylate group, preferably chloro.

## Patentansprüche

1. Verbindung der Formel (I): 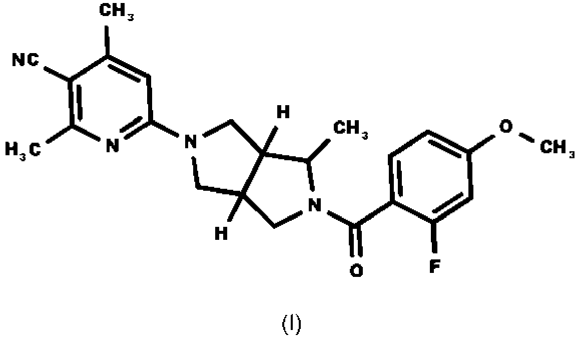 oder ein pharmazeutisch unbedenkliches Salz, Solvat, Hydrat, geometrisches Isomer, Tautomer oder optisches Isomer davon.

2. Verbindung nach Anspruch 1, wobei die zentralen Wasserstoffatome auf der methylierten Octahydropyrrolo[3,4-c]pyrrolgruppe in einer *cis*-Stellung sind.

3. Verbindung nach Anspruch 1 oder 2, wobei die Verbindung von der Formel (I)(a) ist: 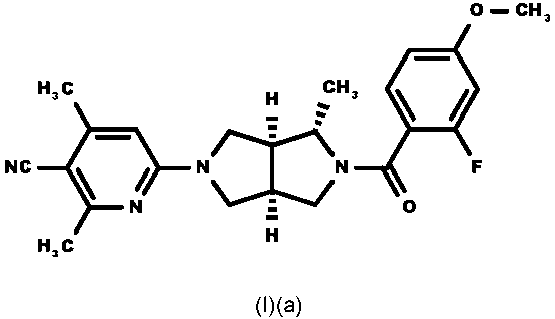 oder ein pharmazeutisch unbedenkliches Salz, Solvat, Hydrat, geometrisches Isomer, Tautomer oder optisches Isomer davon.

4. Pharmazeutische Zusammensetzung, die eine Verbindung nach einem der Ansprüche 1 bis 3 und einen pharmazeutisch unbedenklichen Arzneistoffträger umfasst.

5. Verbindung nach einem der Ansprüche 1 bis 3 oder eine Zusammensetzung nach Anspruch 4 zur Verwendung als ein Arzneimittel.

6. Verbindung nach einem der Ansprüche 1 bis 3 oder eine Zusammensetzung nach Anspruch 4 zur Verwendung bei einem Behandeln einer Erkrankung, einer Störung oder eines Zustands, der durch Orexinrezeptoraktivität vermittelt wird, wobei die Erkrankung, die Störung oder der Zustand aus der Gruppe ausgewählt ist, die aus Folgenden besteht: einer Störung des Schlaf-Wach-Zyklus, einer Stoffwechselstörung, einer neurologische Störung und anderen Störungen wie etwa Ernähren, Trinken, Erregung, Stress, Sucht, insbesondere Drogensucht, Stoffwechsel- und Fortpflanzungsstörungen.

7. Verbindung nach einem der Ansprüche 1 bis 3 oder eine Zusammensetzung nach Anspruch 4 zur Verwendung bei dem Behandeln einer Erkrankung, einer Störung oder eines Zustands, der durch Orexinrezeptoraktivität vermittelt wird, wobei die Erkrankung, die Störung oder der Zustand aus der Gruppe ausgewählt ist, die aus Folgenden besteht: Störungen des Schlaf-Wach-Zyklus, Schlaf-Wach-Übergangsstörung, Insomnie, Syndrom der unruhigen Beine, Jetlag, gestörtem Schlaf, Schlafstörungen infolge neurologischer Störungen, Manien, Depressionen, manischer Depressionen, Schizophrenie, Schmerzsyndrome, Fibromyalgie, neuropathischer Schmerzen, Katatonie, Parkinson-Syndrom, Tourette-Syndrom, Angststörung, Belastungsstörung, Panikstörung, Delir, Demenzen, Alzheimer-Krankheit, Huntington-Krankheit, Übergewicht oder Adipositas, Zustände zugehörig zu Übergewicht oder Adipositas, Insulinresistenz, Diabetes mellitus Typ 2, Hyperlipidämie, Gallenblasensteine, Angina, Hypertonie, Atemnotsyndrom, Tachykardie, Infertilität, Schlafapnoe, Rücken- und Gelenkschmerzen, varikösen Venen, Arthrose, Hypertonie, Tachykardie, kardialen Arrhythmien, Angina pectoris, akuter Herzinsuffizienz, Ulcera, Reizdarmsyndrom, Diarrhoe, gastroösophagealer Refluxkrankheit, Sucht und Alkoholismus.

8. Verbindung nach einem der Ansprüche 1 bis 3 oder eine Zusammensetzung nach Anspruch 4 zur Verwendung bei dem Behandeln einer Erkrankung, einer Störung oder eines Zustands, der durch Orexinrezeptoraktivität vermittelt wird, wobei die Erkrankung, die Störung oder der Zustand eine Störung des Schlaf-Wach-Zyklus, eine Schlaf-Wach-Übergangsstörung oder Insomnie ist.

9. Verwendung einer Verbindung nach einem der Ansprüche 1 bis 3 oder einer Zusammensetzung nach Anspruch 4 bei der Herstellung eines Arzneimittels zum Behandeln einer Erkrankung, einer Störung oder eines Zustands, der durch Orexinrezeptoraktivität vermittelt wird, wobei die Erkrankung, die Störung oder der Zustand aus der Gruppe ausgewählt ist, die aus Folgenden besteht: einer Störung des Schlaf-Wach-Zyklus, einer Stoffwechselstörung, einer neurologische Störung und anderen Störungen wie etwa Ernähren, Trinken, Erregung, Stress, Sucht, insbesondere Drogensucht, Stoffwechsel- und Fortpflanzungsstörungen.

10. Verwendung einer Verbindung nach einem der Ansprüche 1 bis 3 oder einer Zusammensetzung nach Anspruch 4 bei der Herstellung eines Arzneimittels zum Behandeln einer Erkrankung, einer Störung oder eines Zustands, der durch Orexinrezeptoraktivität vermittelt wird, wobei die Erkrankung, die Störung oder der Zustand aus der Gruppe ausgewählt ist, die aus Folgenden besteht: Störungen des Schlaf-Wach-Zyklus, Schlaf-Wach-Übergangsstörung, Insomnie, Syndrom der unruhigen Beine, Jetlag, gestörtem Schlaf, Schlafstörungen infolge neurologischer Störungen, Manien, Depressionen, manischen Depressionen, Schizophrenie, Schmerzsyndrome, Fibromyalgie, neuropathischen Schmerzen, Katatonie, Parkinson-Syndrom, Tourette-Syndrom, Angststörung, Belastungsstörung, Panikstörung, Delir, Demenzen, Alzheimer-Krankheit, Huntington-Krankheit, Übergewicht oder Adipositas, Zustände zugehörig zu Übergewicht oder Adipositas, Insulinresistenz, Diabetes mellitus Typ 2, Hyperlipidämie, Gallenblasensteine, Angina, Hypertonie, Atemnotsyndrom, Tachykardie, Infertilität, Schlafapnoe, Rücken- und Gelenkschmerzen, varikösen Venen, Arthrose, Hypertonie, Tachykardie, kardialen Arrhythmien, Angina pectoris, akuter Herzinsuffizienz, Ulcera, Reizdarmsyndrom, Diarrhoe, gastroösophagealer Refluxkrankheit, Sucht und Alkoholismus.

11. Verwendung einer Verbindung nach einem der Ansprüche 1 bis 3 oder einer Zusammensetzung nach Anspruch 4 bei der Herstellung eines Arzneimittels zum Behandeln einer Erkrankung, einer Störung oder eines Zustands, der durch Orexinrezeptoraktivität vermittelt wird, wobei die Erkrankung, die Störung oder der Zustand eine Störung des Schlaf-Wach-Zyklus, eine Schlaf-Wach-Übergangsstörung oder Insomnie ist.

12. Verfahren zur Herstellung einer Verbindung der Formel (I), das ein Reagieren einer Verbindung der Formel (V) und einer Verbindung der Formel (XVI) umfasst, um die Verbindung der Formel (I) herzustellen: 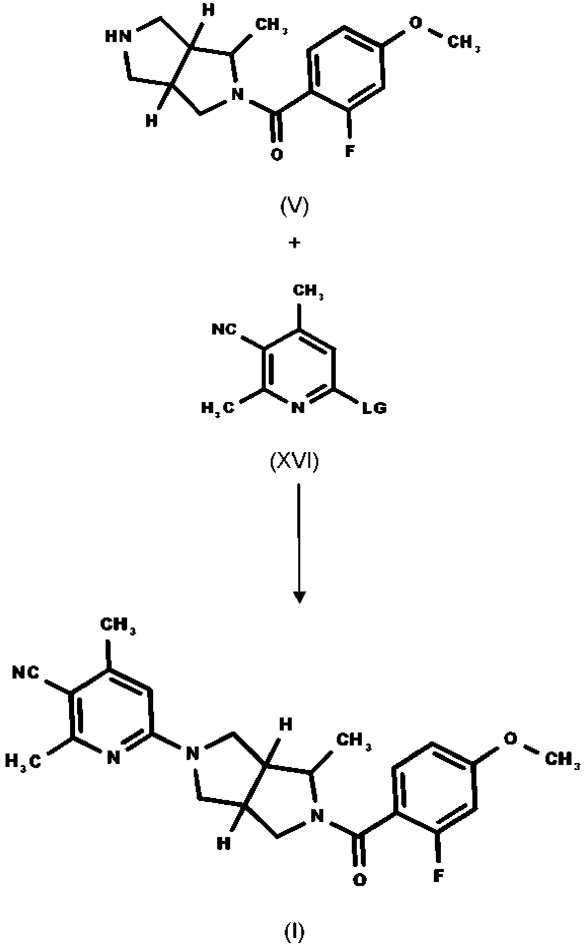 in der LG eine austretende Gruppe wie etwa eine Chlor-, Brom-, Mesylat- oder Tosylatgruppe, vorzugsweise Chlor, ist.

13. Verfahren nach Anspruch 12, wobei die zentralen Wasserstoffatome auf der methylierten Octahydropyrrolo[3,4-c]pyrrolgruppe in Verbindung (V) und Verbindung (I) in einer *cis*-Stellung sind.

14. Verfahren nach Anspruch 12 oder 13, das das Reagieren einer Verbindung der Formel (V)(a) und einer Verbindung der Formel (XVI) umfasst, um eine Verbindung der Formel (I)(a) herzustellen: 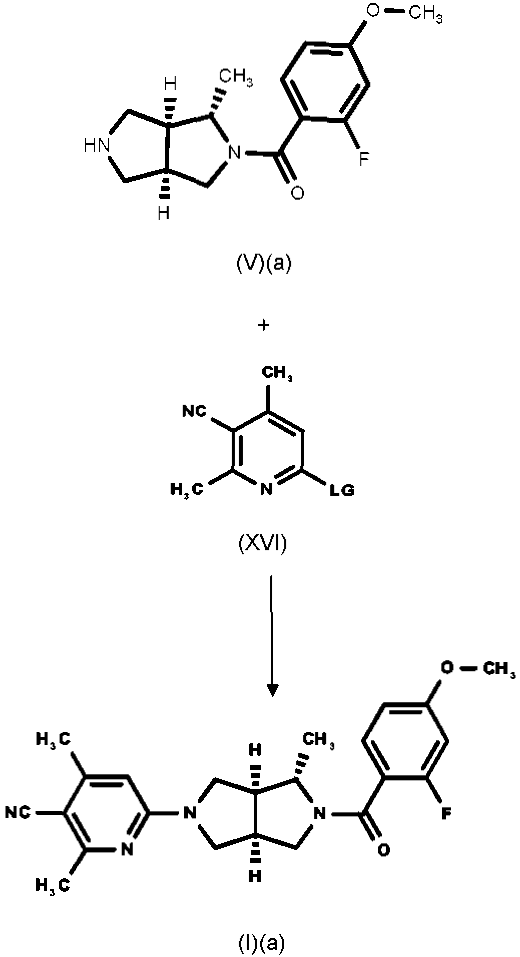 in der LG eine austretende Gruppe wie etwa eine Chlor-, Brom-, Mesylat- oder Tosylatgruppe, vorzugsweise Chlor, ist.

## Revendications

1. Composé de formule (I) : 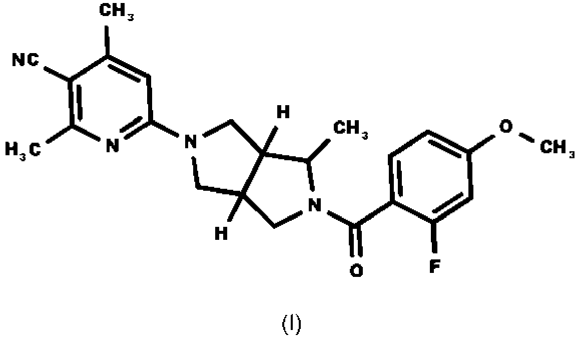 ou un sel pharmaceutiquement acceptable, solvate, hydrate, isomère géométrique, tautomère ou isomère optique de celui-ci.

2. Composé selon la revendication 1, dans lequel les atomes d'hydrogène centraux sur le groupe octahydropyrrolo [3,4-c] pyrrole méthylé sont en position *cis.*

3. Composé selon la revendication 1 ou 2, dans lequel le composé est de formule (I)(a) : 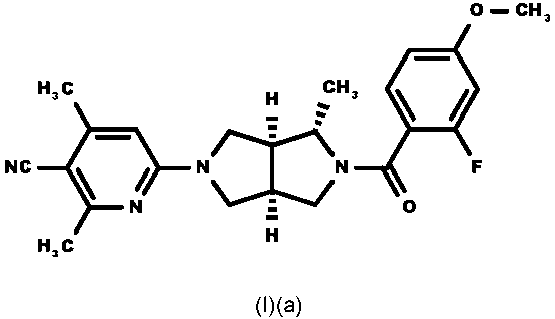 ou un sel pharmaceutiquement acceptable, solvate, hydrate, isomère géométrique, tautomère ou isomère optique de celui-ci.

4. Composition pharmaceutique comprenant un composé selon l'une quelconque des revendications 1 à 3 et un excipient pharmaceutiquement acceptable.

5. Composé selon l'une quelconque des revendications 1 à 3, ou une composition selon la revendication 4, à utiliser comme médicament.

6. Composé selon l'une quelconque des revendications 1 à 3, ou composition selon la revendication 4, à utiliser dans le traitement d'une maladie, d'un trouble ou d'un état médié par l'activité du récepteur de l'orexine, dans lequel la maladie, le trouble ou l'état est choisi dans le groupe constitué par : un trouble du cycle veille-sommeil, un trouble métabolique, un trouble neurologique et d'autres troubles tels que l'alimentation, la consommation d'alcool, l'excitation, le stress, la dépendance, en particulier la toxicomanie, les troubles du métabolisme et de la reproduction.

7. Composé selon l'une quelconque des revendications 1 à 3, ou composition selon la revendication 4, à utiliser dans le traitement d'une maladie, d'un trouble ou d'un état médié par l'activité du récepteur de l'orexine, dans lequel la maladie, le trouble ou l'état est choisi dans le groupe constitué par : les troubles du cycle veille-sommeil, le trouble de transition veille-sommeil, l'insomnie, le syndrome des jambes sans repos, le décalage horaire, le sommeil perturbé, les troubles du sommeil secondaires à des troubles neurologiques, les manies, les dépressions, la maniaco-dépression, la schizophrénie, les syndromes de la douleur, la fibromyalgie, les douleurs neuropathiques, la catatonie, la maladie de Parkinson, le syndrome de Gilles de La Tourette, l'anxiété, le trouble de stress, le trouble panique, le délire, les démences, la maladie d'Alzheimer, la maladie de Huntington, le surpoids ou l'obésité, les conditions liées au surpoids ou à l'obésité, la résistance à l'insuline, le diabète de type II, l'hyperlipidémie, les calculs biliaires, l'angine de poitrine, l'hypertension, l'essoufflement, la tachycardie, la stérilité, l'apnée du sommeil, les douleurs dorsales et articulaires, les varices, l'arthrose, l'hypertension, la tachycardie, les arythmies, l'angine de poitrine, l'insuffisance cardiaque aiguë, les ulcères, le syndrome du côlon irritable, la diarrhée, le reflux gastro-œsophagien, la dépendance et l'alcoolisme.

8. Composé selon l'une quelconque des revendications 1 à 3, ou composition selon la revendication 4, à utiliser dans le traitement d'une maladie, d'un trouble ou d'un état médié par l'activité du récepteur de l'orexine, dans lequel la maladie, le trouble ou l'état est un trouble du cycle veille-sommeil, un trouble de transition veille-sommeil ou une insomnie.

9. Utilisation d'un composé selon l'une quelconque des revendications 1 à 3, ou d'une composition selon la revendication 4, dans la fabrication d'un médicament pour traiter une maladie, un trouble ou une condition médiée par l'activité du récepteur de l'orexine, dans laquelle la maladie, le trouble ou la condition est choisi dans le groupe constitué par :
un trouble du cycle veille-sommeil, un trouble métabolique, un trouble neurologique et d'autres troubles tels que l'alimentation, la consommation d'alcool, l'excitation, le stress, la dépendance, en particulier la toxicomanie, les troubles du métabolisme et de la reproduction.

10. Utilisation d'un composé selon l'une quelconque des revendications 1 à 3, ou d'une composition selon la revendication 4, dans la fabrication d'un médicament pour traiter une maladie, un trouble ou une condition médiée par l'activité du récepteur de l'orexine, dans laquelle la maladie, le trouble ou la condition est choisi dans le groupe constitué par :
les troubles du cycle veille-sommeil, le trouble de transition veille-sommeil, l'insomnie, le syndrome des jambes sans repos, le décalage horaire, le sommeil perturbé, les troubles du sommeil secondaires à des troubles neurologiques, les manies, les dépressions, la maniaco-dépression, la schizophrénie, les syndromes de la douleur, la fibromyalgie, les douleurs neuropathiques, la catatonie, la maladie de Parkinson, le syndrome de Gilles de La Tourette, l'anxiété, le trouble de stress, le trouble panique, le délire, les démences, la maladie d'Alzheimer, la maladie de Huntington, le surpoids ou l'obésité, les conditions liées au surpoids ou à l'obésité, la résistance à l'insuline, le diabète de type II, l'hyperlipidémie, les calculs biliaires, l'angine de poitrine, l'hypertension, l'essoufflement, la tachycardie, la stérilité, l'apnée du sommeil, les douleurs dorsales et articulaires, les varices, l'arthrose, l'hypertension, la tachycardie, les arythmies, l'angine de poitrine, l'insuffisance cardiaque aiguë, les ulcères, le syndrome du côlon irritable, la diarrhée, le reflux gastro-œsophagien, la dépendance et l'alcoolisme.

11. Utilisation d'un composé selon l'une quelconque des revendications 1 à 3, ou d'une composition selon la revendication 4, dans la fabrication d'un médicament pour traiter une maladie, un trouble ou un état médié par l'activité du récepteur de l'orexine, dans laquelle la maladie, le trouble ou l'état est un trouble du cycle veille-sommeil, un trouble de transition veille-sommeil ou une insomnie.

12. Procédé de production d'un composé de formule (I), qui comprend la réaction d'un composé de formule (V) et d'un composé de formule (XVI), pour produire le composé de formule (I) : 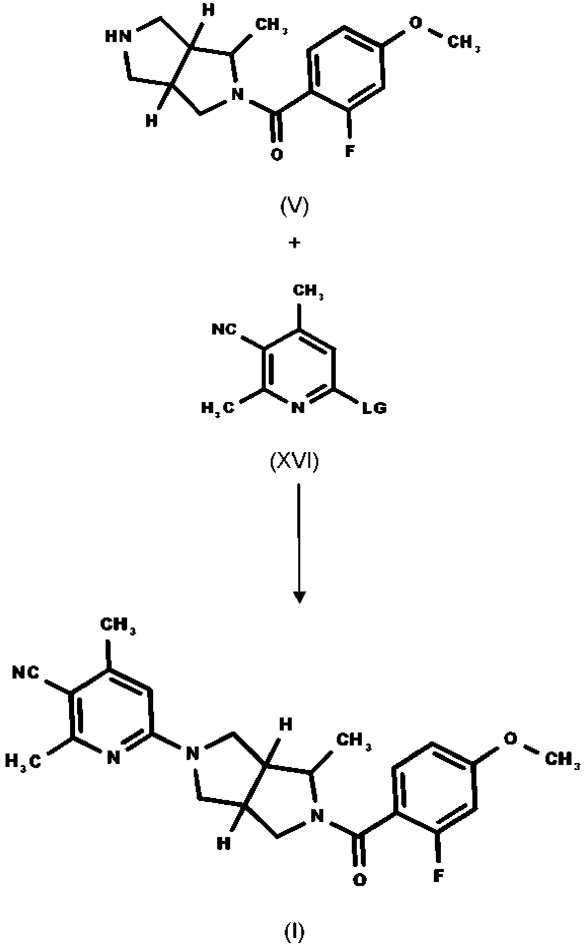 où LG est un groupe partant, tel qu'un groupe chloro, bromo, mésylate ou tosylate, de préférence chloro.

13. Procédé selon la revendication 12, dans lequel les atomes d'hydrogène centraux sur le groupe octahydropyrrolo [3,4-c] pyrrole méthylé dans le composé (V) et le composé (I) sont en position *cis.*

14. Procédé selon la revendication 12 ou 13, qui comprend la réaction d'un composé de formule (V)(a) et d'un composé de formule (XVI), pour produire un composé de formule (I)(a) : 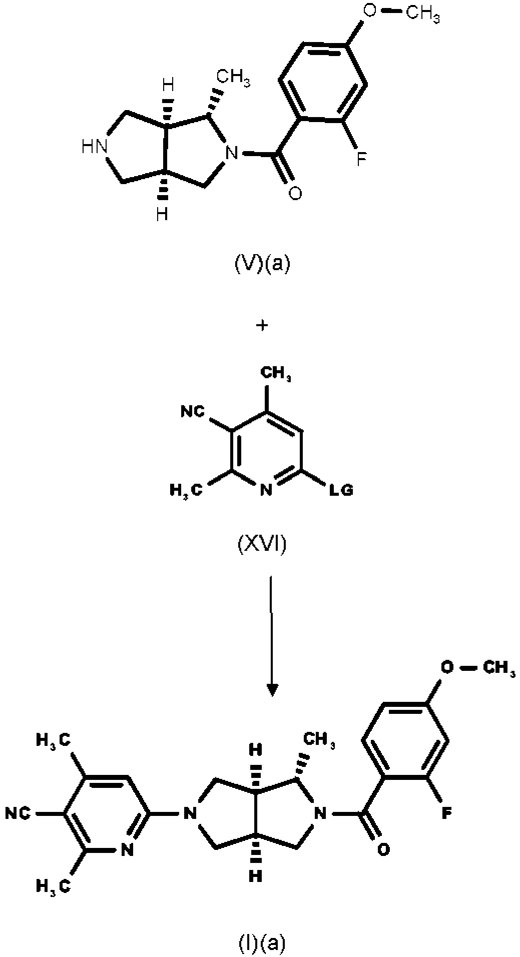 où LG est un groupe partant, tel qu'un groupe chloro, bromo, mésylate ou tosylate, de préférence chloro.
